(19) Europäisches Patentamt
European Patent Office
Office européen des brevets

(11) **EP 3 954 692 A1**

(12) # EUROPEAN PATENT APPLICATION
published in accordance with Art. 153(4) EPC


(43) Date of publication:
**16.02.2022 Bulletin 2022/07**

(21) Application number: **20787011.4**

(22) Date of filing: **30.03.2020**

(51) International Patent Classification (IPC):
***C07D 498/04*** *(2006.01)* ***C07D 213/64*** *(2006.01)*

(52) Cooperative Patent Classification (CPC):
**C07D 213/64; C07D 215/38; C07D 217/22; C07D 401/12; C07D 498/04; C07K 1/113; C07K 1/13**

(86) International application number:
**PCT/CN2020/081983**

(87) International publication number:
**WO 2020/207279 (15.10.2020 Gazette 2020/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2019 CN 201910276925**



(71) Applicant: **Shanghai Institute of Materia Medica, Chinese Academy of Sciences**
**Pudong, Shanghai 201203 (CN)**

(72) Inventors:
- **LI, Bo**
  **Pudong, Shanghai 201203 (CN)**
- **LIU, Peng**
  **Pudong, Shanghai 201203 (CN)**
- **SUN, Haiguo**
  **Pudong, Shanghai 201203 (CN)**
- **XI, Mengyu**
  **Pudong, Shanghai 201203 (CN)**
- **XU, Zhijian**
  **Pudong, Shanghai 201203 (CN)**
- **ZHANG, Yong**
  **Pudong, Shanghai 201203 (CN)**
- **ZHU, Weiliang**
  **Pudong, Shanghai 201203 (CN)**

(74) Representative: **Kador & Partner PartG mbB**
**Corneliusstraße 15**
**80469 München (DE)**


(54) **METHOD FOR MODIFYING AMINO GROUP IN MOLECULE WITH OXAZOLINE QUATERNARY AMMONIUM SALT AND USE THEREOF**


(57) The present invention relates to a method for modifying or marking an amino group in a molecule, wherein the amino group in the molecule is modified or marked with zolinium(s) shown in the following formula 1, wherein the molecule comprises amino acid ester, aminoamide, or peptide/protein. The method has advantages of economy and site selectivity, and has huge potential application value in the field such as pharmaceutical molecular synthesis, probe molecule and diagnostic marker reagent development.


Fig. 1

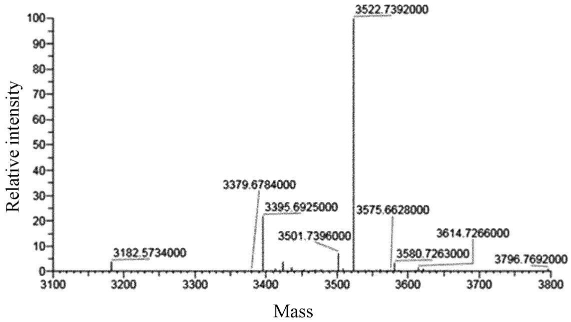


EP 3 954 692 A1

## Description

### Technical field

The application belongs to the field of bioorthogonal chemistry, and specifically, relates to a method for modifying or labeling an amino group in a molecule with the zolinium and use thereof.

### Background

Quaternary ammonium salt is a compound in which all the four hydrogen atoms in the ammonium ion are replaced by hydrocarbyl groups. The four hydrocarbyl groups can be the same or different from each other; and the anion is mostly a halide(F, Cl, Br, I) or an acid radical (such as $HSO_4$-, RCOO-, etc.). Quaternary ammonium compounds are widely used in the fields of medicine and chemical industry, for example, as an antibacterial agent, a disinfectant, a soft antistatic agent, a flocculant, a demulsifier, a drilling fluid, a VES fracturing fluid, a drag reducer, a thickener, an anionic synergist, a phase transfer catalyst, etc.

Among quaternary ammonium salts, an azole quaternary ammonium salt is a very important reagent for organic synthesis. It can undergo 1,3-dipolar cycloaddition reaction, and can be used to prepare an azacarbene compound, or a pharmaceutical intermediate, etc. As an important reagent in organic synthesis, an azole quaternary ammonium salt can undergo reactions such as nucleophilic addition, Michael addition, 1,3-dipolar addition, nucleophilic substitution, or σ-shift rearrangement. In addition, this class of compounds have various physiological activities, for example, 12-methacryloyloxydodecylpyridinium bromide (MDPB) and cetylpyridinium chloride (CPC) can be used as oral disinfectants; 3-alkylpyridine quaternary ammonium salt polymer obtained from marine organisms has activity against lung cancer. Berberine hydrochloride (also known as berberinum), an active ingredient of traditional Chinese medicine, is an isoquinoline alkaloid having an isoquinoline quaternary ammonium salt (also known as benzopyridine quaternary ammonium salt) moiety in its molecular structure, has wide clinical use in treatment of enteritis, bacillary dysentery, etc., and modern studies have revealed that it also has pharmacological effects in the treatment of diseases such as tumors, diabetes, cardiovascular diseases, central nervous system diseases.

Li, Bo et al., initially discovered a new acetal rearrangement reaction and a novel class of N-substituted aryl isoquinolinone compounds with good anti-tumor activity prepared thereby (Eur. J. Med. Chem., 2014, 77: 204-210*)* during previous research on a structural optimization design and synthesis of berberine, an active ingredient of traditional Chinese medicine (Eur. J. Med. Chem., 2013, 70, 677). A novel oxazolinepyridinium quaternary ammonium salt core skeleton was further discovered after in-depth study on the mechanism of the acetal rearrangement reaction, and the core skeleton was applied to click chemistry coupling reaction of amino compounds for the first time, and a series of regioselective reaction products controlled by different nucleophiles were thus obtained (Sci Rep. 2017, 7, 41287*;* CN107759614A*)*. During the process, an oxazoline aryl isoquinoline quaternary ammonium salt compound DCZ0358 with good anti-hematological tumor activity in vivo and in vitro was further discovered (Cancer Lett. 2018, 421: 135-144). However, none of these studies involved the coupling reaction of such zoliniums with amino acid residues.

### Summary of the invention

One technical purpose of the present invention is to provide a method for modifying or labeling an amino group in a molecule.

In one aspect, the present invention provides a method for modifying or labeling an amino group in a molecule, wherein a zolinium represented by the following formula 1 is used to modify or label the amino group in the molecule,

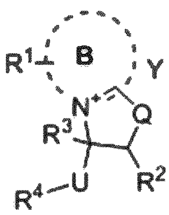

**1**

In formula 1,

Ring B represents a substituted or unsubstituted 5-, 6- or 7-membered nitrogen-containing heterocyclic ring;

Q and U each independently represent oxygen (O), sulfur (S), selenium (Se), tellurium (Te), polonium (Po), nitrogen

(N), phosphorus (P), boron (B) or silicon (Si);
$R^1$ represents 1-5 substituents on the ring B, which are each independently selected from hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, and halogen; alternatively, when $R^1$ represents two or more substituents on the ring B, two adjacent substituents of which may connect to each other together with the atoms on the ring B to form a substituted or unsubstituted aryl, a substituted or unsubstituted 5-7 membered cycloalkyl, a substituted or unsubstituted 5-7 membered heterocycloalkyl containing 1-5 atoms independently selected from oxygen (O), sulfur (S), selenium (Se), tellurium (Te), polonium (Po), nitrogen (N), phosphorus (P), boron (B) and silicon (Si), or a substituted or unsubstituted 5-7 membered heteroaryl containing 1-5 atoms independently selected from oxygen (O), sulfur (S), selenium (Se), tellurium (Te), polonium (Po), nitrogen (N), phosphorus (P), boron (B) and silicon (Si);
$R^2$ and $R^3$ each independently represent hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, or halogen;
$R^4$ represents hydrogen, a substituted or unsubstituted alkyl, or a substituted or unsubstituted aryl;
Y is an acid anion selected from inorganic acid ions, organic acid ions and halide ions, including but not limited to nitrate, sulfate, phosphate, methanesulfonate, benzenesulfonate, acetate, tartarate, citrate, maleate, succinate, salicylate, glycerate, ascorbate, fluoride, chloride, bromide and iodide.

Preferably, in formula 1, ring B is a 5- or 6-membered ring; Q and U are each independently selected from oxygen, sulfur and selenium; $R^1$ represents two or more substituents on ring B, two adjacent substituents of which may connect to each other together with the atoms on the ring B to form a substituted or unsubstituted aryl; $R^2$ and $R^3$ are each independently selected from hydrogen, substituted or unsubstituted alkyl, and substituted or unsubstituted aryl; $R^4$ is a substituted or unsubstituted alkyl.
Specifically, in the method, the molecule includes amino acid esters, amino amides, and peptides/proteins.
Specifically, the method comprises, in the presence or absence of an additive, reacting the zolinium represented by Formula 1 with a molecule containing an amino group in a solvent to prepare a product of which the amino group is labeled or modified.
In an embodiment, the method is carried out by a reaction represented by the following scheme I:

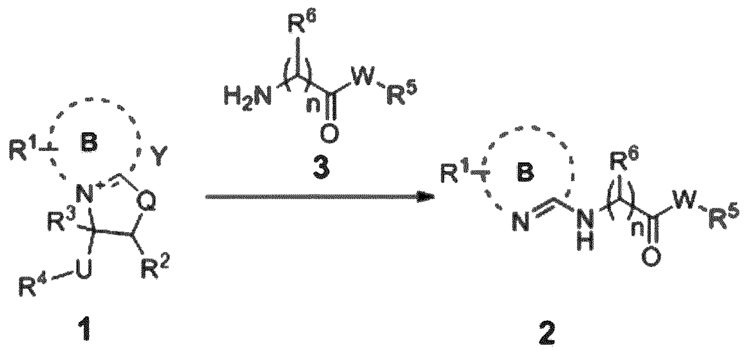

Scheme I

In Scheme I, in the presence or absence of an additive, the zolinium represented by formula 1 reacts with the compound represented by formula 3 in a solvent to prepare a product represented by Formula 2 of which the amino group is modified,

wherein, in formula 3,
n represents an integer of 1-6;
$R^5$ represents hydrogen, substituted or unsubstituted alkyl, or substituted or unsubstituted aryl;

Each of $R^6$ independently represents hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, or halogen; or

$R^5$ and $R^6$ connect to each other to form a 5-7 membered heterocycle containing 1-3 heteroatoms selected from oxygen (O), sulfur (S), selenium (Se), tellurium (Te), polonium (Po), nitrogen (N), phosphorus (P), boron (B) and silicon (Si);
W represents oxygen (O), sulfur (S), selenium (Se), tellurium (Te), polonium (Po), nitrogen (N), phosphorus (P), boron (B), or silicon (Si).
wherein, in formula 2, ring B, $R^1$, $R^5$, $R^6$, W and n are defined as above.

Preferably, in formula 3, n is 1 or 5; W is oxygen or nitrogen; $R^5$ is hydrogen or substituted or unsubstituted alkyl; $R^6$ is amino, substituted or unsubstituted amido, substituted or unsubstituted alkyl, or substituted or unsubstituted phenyl.

Specifically, in the above method, the solvent includes but is not limited to any one or a combination of two or more solvents of aprotic solvents and protic solvents. The aprotic solvent is preferably a solvent which is miscible with water in a ratio of 10% or more (water/solvent), including but not limited to toluene, acetone, dichloromethane, 1,2-dichloroethane, tetrahydrofuran, acetonitrile, xylene, chlorobenzene, dioxane, dimethyl sulfoxide, dimethyl formamide, dimethyl acetamide, etc.; the protic solvent includes but is not limited to n-butanol, n-propanol, ethanol, methanol, glycerol, ethylene glycol, water, etc., preferably, the solvent is selected from n-butanol and water.

Specifically, in the above method, the additive includes, but are not limited to, organic bases or inorganic bases. The organic base includes, but are not limited to, triethylamine, DMAP (N,N-dimethylaniline), DBU (1,8-diazabicycloundec-7-ene), imidazole, pyridine, etc., most preferably is triethylamine; and the inorganic base includes, but are not limited to, potassium carbonate, sodium carbonate, cesium carbonate, sodium bicarbonate, sodium hydroxide, potassium hydroxide, potassium phosphate, sodium phosphate, etc., most preferably is potassium phosphate.

Specifically, in the above method, the reaction is performed at -80°C to 200°C, preferably at 0°C to 50°C, for 0.1 minute to 72 hours.

In another aspect, the present invention provides use of the zolinium represented by the following formula 1 in modifying or labeling an amino group in a molecule,

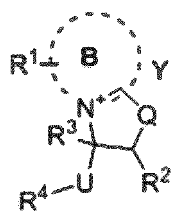

1

wherein, each substituent in Formula 1 is defined as above.

Specifically, in the method, the molecule includes amino acid esters, amino amides, peptides and proteins. In the case of a peptide/protein, the modifying or labeling specifically refers to modifying or labeling $\alpha$-amino group of an amino acid radical, or $\varepsilon$-amino group of a lysine radical in the peptide/protein.

In an embodiment, the modification or labeling is applied in fields of synthesizing pharmaceutical molecules, developing probe molecules and diagnostic labeling reagents, and the like.

## Advantageous effect

It is difficult for a common quaternary ammonium salt in the art, such as a N-acyl pyridine quaternary ammonium salt, a N-oxide pyridine, a N-alkyl pyridine quaternary ammonium salt, etc., to react with an amino acid residue under reaction conditions for the method of the present invention. In contrast, the zolinium described in the present invention can specifically react with an amino acid residue to give a product coupling an amino group at N-ortho position. In addition, when the zolinium of the present invention reacts with an amino group of a complicated peptide molecule, it does not react with the active group such as alcoholic hydroxyl, phenolic hydroxyl, amido, disulfide bond, cyano group and ester group in the structure of the complicated peptide molecule, and thus has good group compatibility. Therefore, the present method for coupling the zolinium with the amino acid residue has economic and site selective advantages, and therefore, it has great application potential in fields of synthesizing pharmaceutical molecules, developing probe molecules and diagnostic labeling reagents, and the like.

## Brief Description of Drawings

Fig. 1 shows a mass spectrum of Compound 2-68.
Fig. 2 shows a mass spectrum of Compound 2-69.
Fig. 3 shows a graph of concentration vs. time of Compound 2-40 in liver tissue.

## Mode for the Invention

### Terms

As used herein, the "substituted" in the term "substituted or unsubstituted" refers to being substituted with one or more substituents selected from halogen, aryl, heteroaryl, alkyl, hydroxyl, mercapto, selanyl, amino and amido.
As used herein, the term "halogen" refers to fluorine, chlorine, bromine or iodine.
As used herein, the term "alkyl" refers to a C1 to C20 linear or branched alkyl, preferably a C1 to C10 linear or branched alkyl, still preferably a C1 to C6 linear or branched alkyl, more preferably methyl, ethyl, n-propyl, isopropyl, n-butyl, isobutyl, or tert-butyl.
As used herein, the term "cycloalkyl" refers to a saturated ring system having 3-7 ring atoms.
As used herein, the term "heterocycloalkyl" refers to a saturated or partially unsaturated ring system containing 1-5 atoms independently selected from oxygen (O), sulfur (S), selenium (Se), tellurium (Te), polonium (Po), nitrogen (N), phosphorus (P), boron (B) and silicon (Si).
As used herein, the term "amido" refers to a C1 to C4 amido, preferably formamido.
As used herein, the term "aryl" refers to a 5-7 membered aryl, preferably phenyl, furyl, thienyl, pyrrolyl, pyridyl, indolyl, quinolinyl, isoquinolinyl, imidazolyl, oxazolyl, thiazolyl, naphthyl, anthryl, or phenanthryl.
As used herein, the term "heteroaryl" refers to a 5-7 membered heteroaryl containing 1-5 heteroatoms selected from oxygen (O), sulfur (S), selenium (Se), tellurium (Te), polonium (Po), nitrogen (N), phosphorus (P), boron (B) and silicon (Si).
In the definition of Ring B, the term "5-, 6- or 7-membered nitrogen-containing heterocyclic ring" includes nitrogen-containing saturated or unsaturated heterocyclic rings, such as pyridine ring, pyrazine ring, triazine ring, oxazole ring, thiazole ring, selenazole ring, imidazole ring, azepine ring, diazepine ring, oxazepine ring, thiazepine ring, and selenazepine ring.
The present invention will be further described below with reference to specific examples, but the present invention is not limited thereto.

### Preparative examples

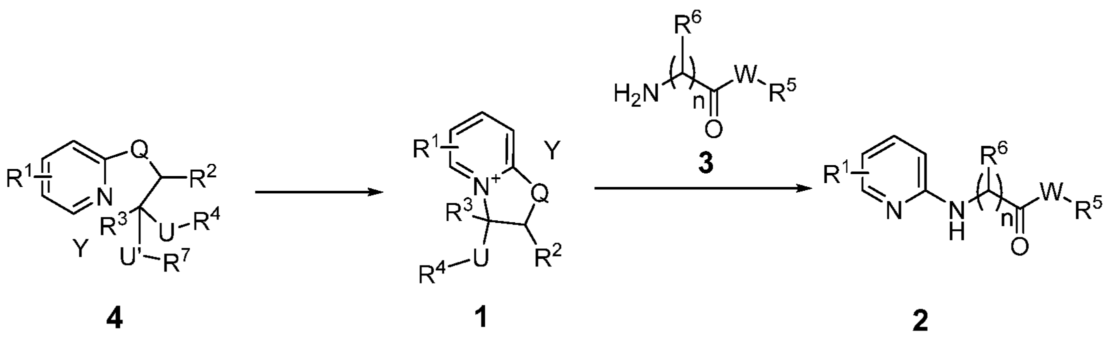

In the above reaction scheme, each substituent is defined as above. In addition, $R^7$ represents hydrogen, a substituted or unsubstituted alkyl, or a substituted or unsubstituted aryl; or $R^7$ connects to $R^4$; U' represents oxygen (O), sulfur (S), selenium (Se), tellurium (Te), polonium (Po), nitrogen (N), phosphorus (P), boron (B) or silicon (Si).
Compound **4** may be prepared following the method in the literature (Li Bo et al., Discovery of N-substituted 3-arylisoquinolone derivatives as antitumor agents originating from O-substituted 3-arylisoquinolines via [2,3] or [3,3] rearrangement , Eur. J. Med. Chem., 2014, 77: 204-210*)*. Specifically, Compound **4** was dissolved in an acid, such as dry hydrochloric acid ether, and the system was reacted overnight while being kept dry. The excess acid, such as the

hydrochloric acid ether, was removed by rotary evaporation to obtain Compound **1.** Then, Compound **1** was added into a solution of Compound **3** in n-butanol or water at room temperature, or Compound **1** was dissolved in n-butanol or water, and then Compound **3** was added thereto. With or without the addition of an additive (such as triethylamine or potassium phosphate, etc), the mixture was stirred overnight. After the reaction was completed, the product **2** was obtained after post-processing steps such as solvent extraction and separation.

**Preparation examples: reactions between a zolinium and various simple amino acid esters or amino amides**

Example 1 Preparation of Compound 2-1

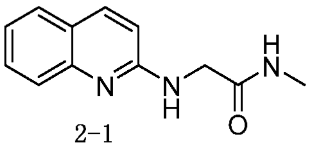

2-1

2-(2,2-dimethoxyethoxy)quinoline, 2-(2,2-dimethoxyethylthio)quinoline or 2-(2,2-dimethoxyethylseleno)quinoline (0.13mmol) was prepared using a substituted or unsubstituted 2-chloroquinoline or 2-chloroisoquinoline compound as a raw material following the method in the literature (Li, Bo et al., Discovery of N-substituted 3-arylisoquinolone derivatives as antitumor agents originating from O-substituted 3-arylisoquinolines via [2,3] or [3,3] rearrangement , Eur. J. Med. Chem., 2014, 77: 204-210*).* Then it was dissolved in dry hydrochloric acid ether (5 ml), and reacted overnight while being kept dry. The excess hydrochloric acid ether was removed by rotary evaporation to obtain an intermediate 1. **a)** the obtained intermediate 1 was directly dissolved in n-butanol or water (10ml), and added with 2-amino-N-methylacetamide hydrochloride (0.26mmol) as an amine nucleophile; or **b)** the obtained intermediate 1 was added to a solution of the amine nucleophile 2-amino-N-methylacetamide hydrochloride (0.26 mmol) in n-butanol or water. With or without addition of triethylamine or potassium phosphate (0.26mmol), the mixture was stirred to complete the reaction, and then directly evaporated off the solvent under reduced pressure to obtain a residue, or extracted with n-butanol prior to evaporating the solvent under reduced pressure. The residue was separated by reversed-phase silica column chromatography to obtain a product 2-1 with a yield of 95%. $^1$H NMR (400 MHz, $CDCl_3$) $\delta$ 7.86 (d, *J*=8.9 Hz, 1H), 7.72 (d, *J*=8.4 Hz, 1H), 7.63 (d, *J*=8.0 Hz, 1H), 7.57 (t, *J*=7.5 Hz, 1H), 7.31-7.25 (m, 1H), 6.96 (s, 1H), 6.71 (d, *J*=8.9 Hz, 1H), 5.67 (s, 1H), 4.24 (s, 2H), 2.84 (d, *J*=4.9 Hz, 3H), 2.24 (s, 1H). $^{13}$C NMR (125 MHz, $CDCl_3$) $\delta$ 171.3, 156.0, 147.4, 137.8, 129.8, 127.5, 126.2, 123.7, 122.8, 111.9, 45.9, 26.2. HRMS (ESI): Calculated for $C_{12}H_{14}N_3O$ $[M+H]^+$: 216.1060, Found: 216.1060.

Example 2 Preparation of Compound **2-2**

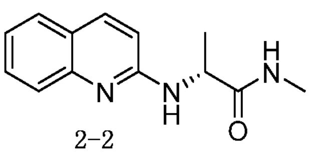

2-2

Compound 2-2 was obtained by the same preparation method in example 1 except that the nucleophile was replaced with (S)-2-amino-N-methylpropionamide hydrochloride, with a yield of 92%. $^1$H NMR (600 MHz, $CDCl_3$) $\delta$ 7.80 (d, *J*=8.8 Hz, 1H), 7.67 (d, *J*=8.3 Hz, 1H), 7.59 (d, *J*=7.9 Hz, 1H), 7.53 (t, *J*=7.3 Hz, 1H), 7.27-7.21 (m, 1H), 7.10 (s, 1H), 6.61 (d, *J*=8.8 Hz, 1H), 5.28 (d, *J*=5.9 Hz, 1H), 4.72 (p, *J*=6.7 Hz, 1H), 2.78 (d, *J*=4.9 Hz, 3H), 1.52 (d, *J*=7.0 Hz, 3H). $^{13}$C NMR (150 MHz, $CDCl_3$) $\delta$ 174.3, 155.8, 147.4, 137.6, 129.7, 127.5, 126.1, 123.6, 122.6, 112.0, 50.7, 26.1, 18.1. HRMS (ESI): Calculated for $C_{13}H_{16}N_3O$ $[M+H]^+$: 230.1288, Found: 230.1288.

Example 3 Preparation of Compound **2-3**

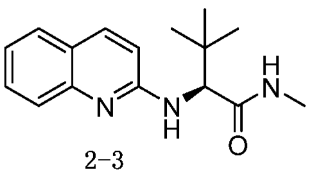

2-3

Compound 2-3 was obtained by the same preparation method in example 1 except that the nucleophile was replaced with 2-amino-N,3,3-trimethylbutanamide hydrochloride, with a yield of 66%. $^1$H NMR (400 MHz, $CDCl_3$) $\delta$ 7.82

(d, *J*=8.9 Hz, 1H), 7.69 (d, *J*=8.4 Hz, 1H), 7.61 (s, 1H), 7.57-7.52 (m, 1H), 7.25 (t, *J*=8.0 Hz, 1H), 6.70 (d, *J*=8.9 Hz, 1H), 6.44 (s, 1H), 5.68 (s, 1H), 4.50 (d, *J*=7.8 Hz, 1H), 2.80 (d, *J*=4.9 Hz, 3H), 1.14 (s, 9H). $^{13}$C NMR (125 MHz, $CDCl_3$) δ 172.6, 156.3, 147.1, 137.8, 129.7, 127.5, 125.7, 123.6, 122.5, 112.0, 34.4, 27.0, 26.1. HRMS (ESI): Calculated for $C_{16}H_{22}N_3O$ $[M+H]^+$: 272.1757, Found: 272.1756.

Example 4 Preparation of Compound **2-4**

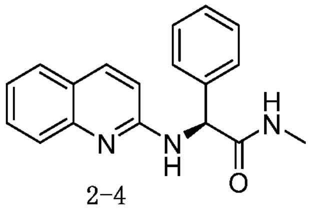

2-4

Compound 2-4 was obtained by the same preparation method in example 1 except that the nucleophile was replaced with (S)-2-amino-N-methyl-2-phenylacetamide hydrochloride, with a yield of 63%. $^{1}$H NMR (400 MHz, $MeOD_4$) δ 7.88 (d, *J*=8.9 Hz, 1H), 7.66-7.60 (m, 2H), 7.57-7.47 (m, 3H), 7.42-7.36 (m, 2H), 7.36-7.30 (m, 1H), 5.69 (s, 1H), 2.77 (s, 3H). $^{13}$C NMR (125 MHz, $MeOD_4$) δ 173.8, 156.1, 147.5, 138.6, 136.8, 128.9, 128.3, 127.7, 127.5, 127.1, 125.6, 123.7, 122.0, 112.4, 59.7, 25.1. HRMS (ESI): Calculated for $C_{18}H_{18}N_3O$ $[M+H]^+$: 292.144, Found: 292.145.

Example 5 Preparation of Compound **2-5**

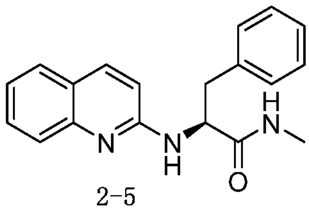

2-5

Compound 2-5 was obtained by the same preparation method in example 1 except that the nucleophile was replaced with (S)-2-amino-N-methyl-2-phenylpropionamide hydrochloride, with a yield of 72%. $^{1}$H NMR (400 MHz, Acetone-$d_6$) δ 7.86 (s, 1H), 7.63 (d, *J*=8.3 Hz, 2H), 7.50 (t, *J*=7.5 Hz, 1H), 7.41 (s, 1H), 7.33 (d, *J*=7.5 Hz, 2H), 7.25 (t, *J*=7.5 Hz, 2H), 7.17 (s, 2H), 6.88 (d, *J*=8.9 Hz, 1H), 6.50 (s, 1H), 5.11-5.02 (m, 1H), 3.30 (dd, *J*=13.8, 5.9 Hz, 1H), 3.13 (dd, *J*=13.7, 7.8 Hz, 1H), 2.70 (d, *J*=4.7 Hz, 3H). $^{13}$C NMR (125 MHz, Acetone-$d_6$) δ 205.2, 172.3, 156.2, 147.8, 138.5, 136.7, 129.3, 129.0, 128.1, 127.4, 126.2, 126.1, 123.6, 121.8, 113.1, 56.0, 38.0, 25.2. HRMS (ESI): Calculated for $C_{19}H_{20}N_3O$ $[M+H]^+$: 306.1601, Found: 306.1597.

Example 6 Preparation of Compound **2-6**

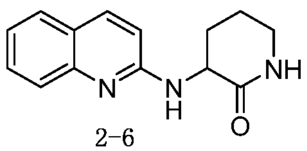

2-6

Compound 2-6 was obtained by the same preparation method in example 1 except that the nucleophile was replaced with 3-aminopiperidin-2-one hydrochloride, with a yield of 28%. $^{1}$H NMR (500 MHz, $CDCl_3$) δ 7.76 (d, *J*=8.8 Hz, 1H), 7.69 (d, *J*=8.3 Hz, 1H), 7.57 (d, *J*=7.9 Hz, 1H), 7.55-7.49 (m, 1H), 7.21 (t, *J*=7.4 Hz, 1H), 6.71 (d, *J*=8.8 Hz, 1H), 6.34 (s, 0H), 5.86 (s, 0H), 4.60 (dt, *J*=11.3, 6.0 Hz, 2H), 3.39 (dd, *J*=7.3, 4.2 Hz, 2H), 2.90 (dd, *J*=12.4, 5.0 Hz, 2H), 2.02 (dddt, *J*=33.9, 13.7, 9.0, 4.5 Hz, 2H), 1.66 (qd, *J*=11.7, 3.4 Hz, 1H). $^{13}$C NMR (125 MHz, $CDCl_3$) δ 173.2, 156.1, 147.8, 136.8, 129.2, 127.4, 126.5, 123.6, 122.2, 113.1, 52.0, 41.8, 27.5, 21.2. HRMS (ESI): Calculated for $C_{14}H_{16}N_3O$ $[M+H]^+$: 242.1288, Found: 242.1286.

Example 7 Preparation of Compound 2-7

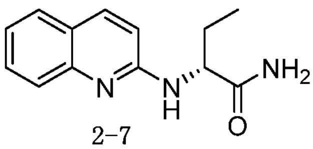

2-7

Compound 2-7 was obtained by the same preparation method in example 1 except that the nucleophile was replaced with 2-aminobutanamide hydrochloride, with a yield of 85%. $^1$H NMR (400 MHz, DMSO-$d_6$) δ 7.85 (d, *J*=8.9 Hz, 1H), 7.62 (d, *J*=7.9 Hz, 1H), 7.48-7.43 (m, 3H), 7.15 (ddd, *J*=8.1, 4.8, 3.2 Hz, 1H), 7.06 (d, *J*=7.8 Hz, 1H), 6.99 (s, 1H), 6.93 (d, *J*=8.9 Hz, 1H), 4.54 (q, *J*=6.9 Hz, 1H), 1.90-1.76 (m, 1H), 1.76-1.63 (m, 1H), 0.94 (t, *J*=7.4 Hz, 3H). $^{13}$C NMR (125 MHz, DMSO-$d_6$) δ 175.1, 157.0, 148.0, 136.6, 129.4, 127.9, 126.0, 123.5, 121.7, 113.9, 55.4, 25.8, 10.8. HRMS (ESI): Calculated for $C_{13}H_{16}N_3O$ $[M+H]^+$: 230.1288, Found: 230.1288.

Example 8 Preparation of Compound 2-8

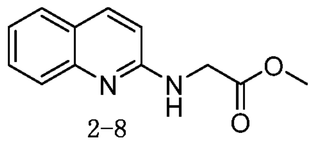

2-8

Compound 2-8 was obtained by the same preparation method in example 1 except that the nucleophile was replaced with glycine methyl ester hydrochloride, with a yield of 95%. $^1$H NMR (400 MHz, $CDCl_3$) δ 7.83 (d, *J*=8.9 Hz, 1H), 7.71 (d, *J*=8.4 Hz, 1H), 7.60 (d, *J*=7.9 Hz, 1H), 7.57-7.51 (m, 1H), 7.23 (t, 1H), 6.72 (d, 1H), 5.24 (s, 1H), 4.38 (d, *J*=5.1 Hz, 2H), 3.80 (s, 3H). $^{13}$C NMR (125 MHz, $CDCl_3$) δ 171.8, 155.7, 147.6, 137.4, 129.5, 127.4, 126.4, 123.7, 122.5, 112.1, 52.3, 43.3, 29.7. HRMS (ESI): Calculated for $C_{12}H_{13}N_2O_2$ $[M+H]^+$: 217.0973, Found: 217.0972.

Example 9 Preparation of Compound 2-9

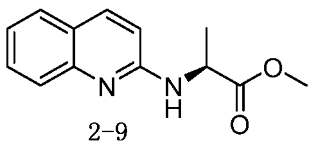

2-9

Compound 2-9 was obtained by the same preparation method in example 1 except that the nucleophile was replaced with D-alanine methyl ester hydrochloride, with a yield of 93%. $^1$H NMR (400 MHz, $CDCl_3$) δ 7.81 (d, *J*=8.8 Hz, 1H), 7.68 (d, *J*=8.3 Hz, 1H), 7.59 (d, *J*=8.8 Hz, 1H), 7.53 (t, *J*=7.7 Hz, 1H), 7.25-7.21 (m, 1H), 6.67 (d, *J*=8.8 Hz, 1H), 5.27 (s, 1H), 4.90 (p, *J*=7.1 Hz, 1H), 3.77 (s, 3H), 1.56 (d, *J*=7.1 Hz, 3H). $^{13}$C NMR (125 MHz, $CDCl_3$) δ 175.0, 155.3, 147.4, 137.4, 129.5, 127.4, 126.4, 123.6, 122.5, 112.0, 52.3, 49.7, 18.6. HRMS (ESI): Calculated for $C_{13}H_{15}N_2O_2$ $[M+H]^+$: 231.1128, Found: 231.1129.

Example 10 Preparation of Compound **2-10**

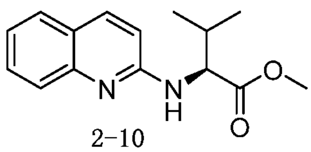

2-10

Compound 2-10 was obtained by the same preparation method in example 1 except that the nucleophile was replaced with L-valine methyl ester hydrochloride, with a yield of 91%. $^1$H NMR (400 MHz, $CDCl_3$) δ 7.76 (d, *J*=8.8 Hz, 1H), 7.67 (d, *J*=8.4 Hz, 1H), 7.56 (d, *J*=8.8 Hz, 1H), 7.54-7.49 (m, 1H), 7.24-7.19 (m, 1H), 6.67 (d, *J*=8.8 Hz, 1H), 5.21 (d, *J*=7.5 Hz, 1H), 4.87 (dd, *J*=8.3, 5.4 Hz, 1H), 3.75 (s, 3H), 1.05 (dd, *J*=6.8, 3.4 Hz, 6H). $^{13}$C NMR (125 MHz, $CDCl_3$) δ 174.1, 156.1, 147.7, 137.2, 129.4, 127.3, 126.5, 123.7, 122.3, 112.1, 59.0, 51.9, 31.3, 19.1, 18.5. HRMS (ESI): Calculated for $C_{13}H_{15}N_2O_2$ $[M+H]^+$: 231.1128, Found: 231.1129.

Example 11 Preparation of Compound **2-11**

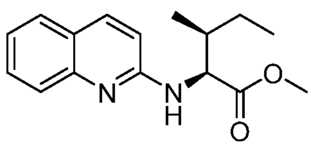

2-11

Compound 2-11 was obtained by the same preparation method in example 1 except that the nucleophile was replaced with L-isoleucine methyl ester hydrochloride, with a yield of 82%. $^1$H NMR (400 MHz, $CDCl_3$) δ 7.83 (d, *J*=8.8

Hz, 1H), 7.69 (d, *J*=8.4 Hz, 1H), 7.60 (d, *J*=7.9 Hz, 1H), 7.54 (t, *J*=7.1 Hz, 1H), 7.24 (t, *J*=7.4 Hz, 1H), 6.70 (dd, *J*=8.9, 4.5 Hz, 1H), 5.11 (s, 1H), 4.93 (dd, *J*=8.3, 5.5 Hz, 1H), 3.77 (s, 3H), 2.10-2.00 (m, 1H), 1.71-1.54 (m, 2H), 1.05-1.00 (m, 6H). HRMS (ESI): Calculated for $C_{16}H_{21}N_2O_2$ $[M+H]^+$: 273.1598, Found: 273.1603.

Example 12 Preparation of Compound **2-12**

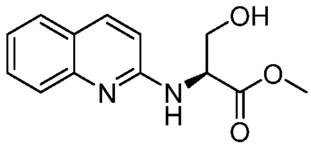

2-12

Compound 2-12 was obtained by the same preparation method in example 1 except that the nucleophile was replaced with L-serine methyl ester hydrochloride, with a yield of 88%. $^1H$ NMR (400 MHz, $CDCl_3$) δ 7.86 (d, J=8.9 Hz, 1H), 7.68 (d, J=8.4 Hz, 1H), 7.62 (d, J=7.9 Hz, 1H), 7.59-7.53 (m, 1H), 7.28-7.25 (m, 1H), 6.77 (d, J=8.9 Hz, 1H), 5.87 (s, 1H), 4.97 (s, 1H), 4.26 (dd, J=10.9, 2.6 Hz, 1H), 3.98 (dd, J=10.9, 6.2 Hz, 1H), 3.85 (s, 3H). $^{13}C$ NMR (150 MHz, $CDCl_3$) δ 171.8, 155.6, 146.2, 138.2, 130.0, 127.5, 125.6, 123.5, 123.0, 112.5, 65.5, 58.0, 52.9. HRMS (ESI): Calculated for $C_{13}H_{15}N_2O_3$ $[M+H]^+$: 247.1081, Found: 247.1077.

Example 13 Preparation of Compound **2-13**

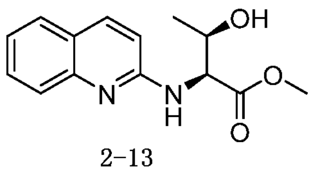

2-13

Compound 2-13 was obtained by the same preparation method in example 1 except that the nucleophile was replaced with D-threonine methyl ester hydrochloride, with a yield of 66%. $^1H$ NMR (400 MHz, $CDCl_3$) δ 7.86 (d, J=8.8 Hz, 1H), 7.68 (d, J=8.3 Hz, 1H), 7.62 (dd, J=8.0, 1.2 Hz, 1H), 7.55 (ddd, J=8.4, 7.0, 1.5 Hz, 1H), 7.28-7.24 (m, 1H), 6.77 (d, J=8.8 Hz, 1H), 5.51 (s, 1H), 4.96 (dd, J=7.3, 3.8 Hz, 1H), 4.40 (qd, J=6.4, 3.7 Hz, 1H), 3.82 (s, 3H), 1.35 (d, J=6.4 Hz, 3H). $^{13}C$ NMR (150 MHz, $CDCl_3$) δ 172.8, 156.0, 147.1, 137.6, 129.6, 127.4, 126.3, 123.8, 122.7, 112.4, 69.3, 59.9, 52.5, 20.4. HRMS (ESI): Calculated for $C_{14}H_{17}N_2O_3$ $[M+H]^+$: 261.1234, Found: 261.1228.

Example 14 Preparation of Compound **2-14**

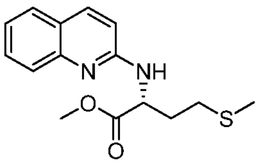

2-14

Compound 2-14 was obtained by the same preparation method in example 1 except that the nucleophile was replaced with L-methionine methyl ester hydrochloride, with a yield of 96%. $^1H$ NMR (400 MHz, $CDCl_3$) δ 7.82 (d, *J*=8.8 Hz, 1H), 7.68 (d, *J*=8.4 Hz, 1H), 7.59 (d, *J*=7.9 Hz, 1H), 7.53 (t, *J*=7.7 Hz, 1H), 7.24 (t, *J*=7.4 Hz, 1H), 6.70 (d, *J*=8.8 Hz, 1H), 5.42 (s, 1H), 5.06 (q, *J*=7.2 Hz, 1H), 3.78 (s, 3H), 2.65 (td, *J*=7.9, 3.4 Hz, 2H), 2.35 (dq, *J*=13.5, 7.4 Hz, 1H), 2.16 (dd, *J*=14.3, 7.5 Hz, 1H). $^{13}C$ NMR (125 MHz, $CDCl_3$) δ 173.9, 155.4, 147.3, 137.5, 129.6, 127.4, 126.4, 123.7, 122.6, 112.0, 53.2, 52.4, 32.0, 30.3, 15.5. HRMS (ESI): Calculated for $C_{15}H_{19}N_2O_2S$ $[M+H]^+$: 291.1162, Found: 291.1167.

Example 15 Preparation of Compound **2-15**

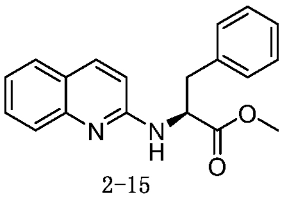

2-15

Compound 2-15 was obtained by the same preparation method in example 1 except that the nucleophile was replaced with L-phenylalanine methyl ester hydrochloride, with a yield of 61%. $^1$H NMR (400 MHz, $CDCl_3$) δ 7.82 (dd, *J*=8.8, 2.4 Hz, 1H), 7.75 (d, *J*=8.1 Hz, 1H), 7.62 (d, *J*=7.7 Hz, 1H), 7.60-7.54 (m, 1H), 7.35-7.26 (m, 4H), 7.21 (d, *J*=7.3 Hz, 2H), 6.65 (dd, *J*=8.8, 2.7 Hz, 1H), 5.27-5.21 (m, 1H), 5.19 (s, 1H), 3.77 (d, *J*=2.8 Hz, 3H), 3.39 (ddd, *J*=13.7, 5.5, 2.4 Hz, 1H), 3.27 (ddd, *J*=13.9, 5.7, 2.5 Hz, 1H). $^{13}$C NMR (125 MHz, $CDCl_3$) δ 173.3, 155.1, 147.1, 137.6, 136.6, 130.4, 129.6, 129.4, 128.5, 127.4, 126.9, 126.2, 123.6, 122.6, 112.1, 55.1, 52.2. HRMS (ESI): Calculated for $C_{19}H_{19}N_2O_2$ $[M+H]^+$: 307.1441, Found: 307.1437.

Example 16 Preparation of Compound **2-16**

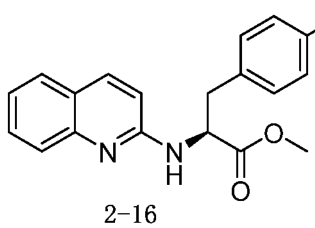

2-16

Compound 2-16 was obtained by the same preparation method in example 1 except that the nucleophile was replaced with L-tyrosine methyl ester hydrochloride, with a yield of 63%. $^1$H NMR (400 MHz, $CDCl_3$) δ 7.85 (d, *J*=8.9 Hz, 1H), 7.73 (d, *J*=8.5 Hz, 1H), 7.61 (dd, *J*=8.0, 1.2 Hz, 1H), 7.54 (ddd, *J*=8.4, 7.0, 1.5 Hz, 1H), 7.26 (ddd, *J*=8.0, 7.0, 1.1 Hz, 1H), 6.97 (d, *J*=8.5 Hz, 2H), 6.68 (d, *J*=8.5 Hz, 2H), 6.64 (d, *J*=8.9 Hz, 1H), 5.47 (s, 1H), 5.03 (s, 1H), 3.75 (s, 3H), 3.25 (dd, *J*=13.9, 5.3 Hz, 1H), 3.13 (dd, *J*=13.9, 6.4 Hz, 1H). $^{13}$C NMR (125 MHz, $CDCl_3$) δ 173.6, 155.5, 155.5, 147.0, 138.1, 130.4, 129.9, 127.5, 127.3, 125.8, 123.6, 122.7, 115.6, 111.5, 55.5, 52.3, 37.2. HRMS (ESI): Calculated for $C_{19}H_{19}N_2O_3$ $[M+H]^+$: 323.139, Found: 323.1388.

Example 17 Preparation of Compound **2-17**

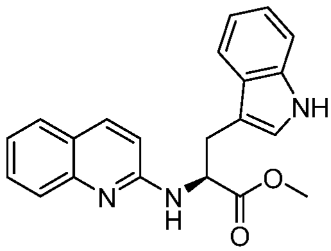

2-17

Compound 2-17 was obtained by the same preparation method in example 1 except that the nucleophile was replaced with L-tryptophan methyl ester hydrochloride, with a yield of 70%. $^1$H NMR (400 MHz, $CDCl_3$) δ 8.27 (s, 1H), 7.80 (d, *J*=8.9 Hz, 1H), 7.76 (d, *J*=8.4 Hz, 1H), 7.61 (d, *J*=7.9 Hz, 2H), 7.59-7.54 (m, 1H), 7.35 (d, *J*=8.1 Hz, 1H), 7.28-7.24 (m, 1H), 7.20 (t, *J*=7.1 Hz, 1H), 7.13 (t, *J*=7.5 Hz, 1H), 7.01 (d, *J*=2.1 Hz, 1H), 6.57 (d, *J*=8.8 Hz, 1H), 5.34-5.29 (m, 1H), 5.27 (d, *J*=8.2 Hz, 1H), 3.73 (s, 3H), 3.54 (dd, *J*=14.6, 5.2 Hz, 1H), 3.45 (dd, *J*=14.6, 5.6 Hz, 1H). $^{13}$C NMR (125 MHz, $CDCl_3$) δ 173.9, 155.4, 147.5, 137.4, 136.2, 129.5, 127.8, 127.4, 126.4, 123.6, 122.9, 122.5, 122.1, 119.6, 118.8, 112.2, 111.2, 110.7, 54.6, 52.2, 27.9. HRMS (ESI): Calculated for $C_{21}H_{20}N_3O_2$ $[M+H]^+$: 346.155, Found: 346.156.

Example 18 Preparation of Compound **2-18**

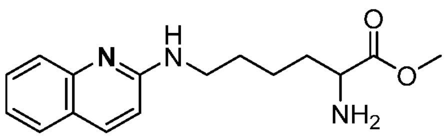

2-18

Compound 2-18 was obtained by the same preparation method in example 1 except that the nucleophile was replaced with lysine methyl ester hydrochloride, with a yield of 53%. $^1$H NMR (400 MHz, $D_2O$) δ 7.94 (s, 1H), 7.63 (t, 1H), 7.59 (d, *J*=7.5 Hz, 1H), 7.53 (s, 1H), 7.35 (t, *J*=7.6 Hz, 1H), 6.76 (s, 1H), 4.06 (t, *J*=6.4 Hz, 1H), 3.70 (s, 3H), 3.33 (t, *J*=6.7 Hz, 2H), 2.00-1.80 (m, 2H), 1.67 (p, *J*=7.2 Hz, 2H), 1.55-1.34 (m, 2H). $^{13}$C NMR (125 MHz, $D_2O$) δ 170.6, 152.5,

141.9, 135.6, 132.5, 128.7, 125.4, 121.1, 117.0, 113.8, 53.6, 52.7, 41.6, 29.4, 26.9, 21.7. HRMS (ESI): Calculated for $C_{6}H_{22}N_{3}O_{2}$ $[M+H]^+$: 288.1707, Found: 288.17.

Example 19 Preparation of Compound 2-19

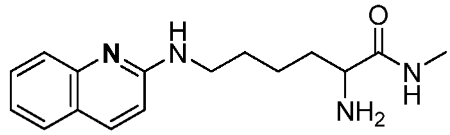

2-19

Compound 2-19 was obtained by the same preparation method in example 1 except that the nucleophile was replaced with lysine formamide, with a yield of 80%. $^1$H NMR (500 MHz, Chloroform-*d*) δ 7.80 (d, *J*=8.8 Hz, 1H), 7.65 (d, *J*=8.2 Hz, 1H), 7.57 (dd, *J*=8.0, 1.4 Hz, 1H), 7.51 (ddd, *J*=8.4, 6.9, 1.5 Hz, 1H), 7.22-7.17 (m, 1H), 6.63 (d, *J*=8.9 Hz, 1H), 3.56-3.49 (m, 2H), 3.40-3.33 (m, 1H), 2.80 (d, *J*=4.9 Hz, 3H), 1.73-1.58 (m, 6H).

Example 20 Preparation of Compound **2-20**

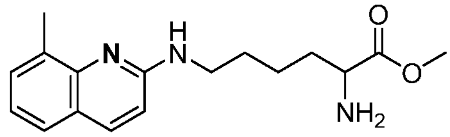

**2-20**

Compound 2-20 was obtained by the same preparation method in example 1 except that 2-(2,2-dimethoxyethoxy)quinoline was replaced with 2-(2,2-dimethoxyethoxy)-8-methylquinoline, and the nucleophile was replaced with lysine methyl ester hydrochloride, with a yield of 56%. $^1$H NMR (400 MHz, $MeOD_4$) δ 8.44 (s, 1H), 7.77 (d, *J*=7.9 Hz, 1H), 7.68 (d, *J*=7.5 Hz, 1H), 7.44 (t, *J*=7.7 Hz, 1H), 7.31 (s, 1H), 4.10 (t, *J*=6.4 Hz, 1H), 3.86 (s, 3H), 3.63 (t, *J*=8.1 Hz, 2H), 2.65 (s, 3H), 2.11-1.93 (m, 2H), 1.81 (ddd, *J*=28.3, 14.9, 6.4 Hz, 2H), 1.71-1.55 (m, 2H). $^{13}$C NMR (125 MHz, MeOD4) δ 169.2, 161.5, 153.5, 144.8, 134.0, 126.5, 124.7, 121.0, 108.7, 52.0, 52.0, 41.6, 29.4, 21.5, 15.1. HRMS (ESI): Calculated for $C_{17}H_{24}N_3O_2$ $[M+H]^+$: 302.1863, Found: 302.1867.

Example 21 Preparation of Compound **2-21**

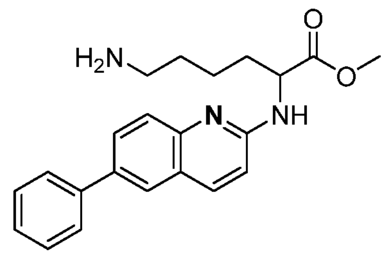

**2-21**

Compound 2-21 was obtained by the same preparation method in example 1 except that 2-(2,2-dimethoxyethoxy)quinoline was replaced with 2-(2,2-dimethoxyethoxy)-7-phenylquinoline, and the nucleophile was replaced with lysine methyl ester hydrochloride, with a yield of 40%. $^1$H NMR (400 MHz, $MeOD_4$) δ 8.42 (s, 1H), 8.15 (s, 1H), 8.10 (d, *J*=9.5 Hz, 1H), 7.95 (s, 1H), 7.73 (d, *J*=7.3 Hz, 2H), 7.51 (t, *J*=7.6 Hz, 2H), 7.42 (t, *J*=7.4 Hz, 1H), 7.25 (d, *J*=9.4 Hz, 1H), 3.84 (s, 3H), 2.99 (t, *J*=7.4 Hz, 2H), 2.28-2.16 (m, 1H), 2.10-1.97 (m, 1H), 1.84-1.72 (m, 2H), 1.64 (dt, *J*=16.6, 8.5 Hz, 2H). $^{13}$C NMR (125 MHz, MeOD4) δ 170.2, 161.2, 153.0, 138.5, 135.2, 131.3, 128.5, 127.5, 126.4, 125.8, 121.8, 117.6, 54.6, 51.8, 38.6, 30.3, 26.3, 22.0. HRMS (ESI): Calculated for $C_{22}H_{26}N_3O_2$ $[M+H]^+$: 364.202, Found: 364.2024.

Example 22 Preparation of Compound **2-22**

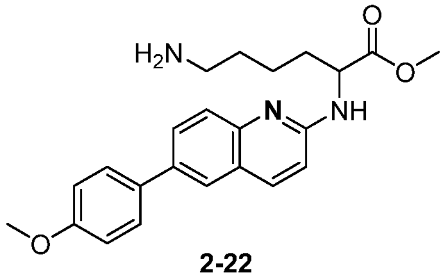

**2-22**

Compound 2-22 was obtained by the same preparation method in example 1 except that 2-(2,2-dimethoxyethoxy)quinoline was replaced with 2-(2,2-dimethoxyethoxy)-6-(4-methoxyphenyl)quinoline, and the nucleophile was replaced with lysine methyl ester hydrochloride, with a yield of 42%. $^1H$ NMR (400 MHz, $MeOD_4$) δ 8.39 (s, 1H), 8.05 (d, *J*=11.0 Hz, 2H), 7.91 (s, 1H), 7.66 (d, *J*=8.8 Hz, 2H), 7.23 (d, *J*=9.4 Hz, 1H), 7.05 (d, *J*=8.8 Hz, 2H), 3.86 (s, 3H), 3.84 (s, 3H), 2.98 (t, *J*=7.5 Hz, 2H), 2.20 (s, 1H), 2.02 (s, 1H), 1.82-1.73 (m, 2H), 1.63 (dt, *J*=14.2, 7.2 Hz, 2H). $^{13}C$ NMR (125 MHz, MeOD4) δ 170.2, 159.7, 152.8, 138.2, 134.7, 130.9, 130.8, 127.5, 127.4, 124.9, 121.8, 117.5, 113.9, 54.6, 54.1, 51.8, 38.6, 30.3, 26.3, 22.0. HRMS (ESI): Calculated for $C_{23}H_{28}N_3O_3$ $[M+H]^+$: 394.2125, Found: 394.2122.

Example 23 Preparation of Compound 2-23

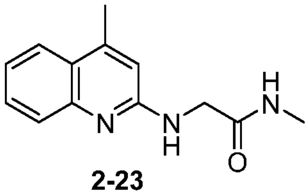

**2-23**

Compound 2-23 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-4-methylquinoline or 2-(2,2-dimethoxyethylthio)-4-methylquinoline, with a yield of 91%. $^1H$ NMR (400 MHz, $CDCl_3$) δ 7.81 (s, 1H), 7.73 (s, 1H), 7.58 (s, 1H), 7.30 (s, 1H), 7.05 (s, 1H), 6.55 (s, 1H), 5.63 (s, 1H), 4.21 (s, 2H), 2.83 (d, *J*=4.5 Hz, 3H), 2.56 (s, 3H). $^{13}C$ NMR (125 MHz, DMSO-$d_6$) δ 170.9, 156.7, 147.8, 144.1, 129.3, 126.4, 124.2, 123.8, 121.9, 113.5, 44.3, 26.0, 18.7. HRMS (ESI) : Calculated for $C_{13}H_{16}N_3O$ $[M+H]^+$: 230.1288, Found: 230.1291.

Example 24 Preparation of Compound **2-24**

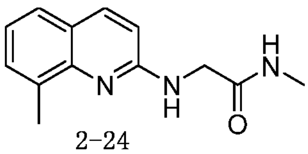

2-24

Compound 2-24 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-8-methylquinoline or 2-(2,2-dimethoxyethylthio)-8-methylquinoline, with a yield of 45%. $^1H$ NMR (400 MHz, $CDCl_3$) δ 7.85 (d, *J*=8.8 Hz, 1H), 7.50 (dd, *J*=8.0, 1.4 Hz, 1H), 7.46 (d, *J*=7.1 Hz, 0H), 7.24 (s, 2H), 7.19 (dd, *J*=7.9, 7.1 Hz, 1H), 6.72 (d, *J*=8.8 Hz, 1H), 5.50 (s, 1H), 4.22 (d, *J*=5.2 Hz, 2H), 2.83 (d, *J*=4.9 Hz, 3H), 2.65 (s, 3H). $^{13}C$ NMR (125 MHz, $CDCl_3$) δ 171.9, 155.2, 146.1, 138.0, 134.0, 130.1, 125.5, 123.4, 122.5, 111.6, 46.5, 26.0, 17.9. HRMS (ESI) : Calculated for $C_{13}H_{16}N_3O$ $[M+H]^+$: 230.1288, Found: 230.1291.

Example 25 Preparation of Compound **2-25**

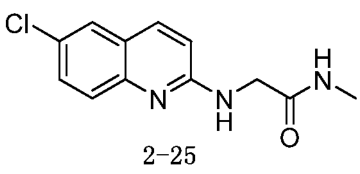

2-25

Compound 2-25 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-6-chloroquinoline or 2-(2,2-dimethoxyethylthio)-6-chloroquinoline, with a yield of 53%. $^1$H NMR (400 MHz, MeOD4) δ 7.86 (d, *J*=9.0 Hz, 1H), 7.65 (s, 1H), 7.59 (d, *J*=9.0 Hz, 1H), 7.46 (d, *J*=8.9 Hz, 1H), 6.88 (d, *J*=8.8 Hz, 1H), 4.13 (s, 2H), 2.76 (s, 3H). $^{13}$C NMR (125 MHz, DMSO-*d*$_6$) δ 170.6, 157.3, 146.6, 135.9, 129.6, 128.0, 126.6, 125.6, 124.4, 114.9, 44.3, 26.0. HRMS (ESI): Calculated for $C_{12}H_{13}ClN_3O$ [M+H]$^+$: 250.0742, Found: 250.0739.

Example 26 Preparation of Compound **2-26**

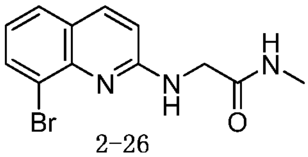

2-26

Compound 2-26 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-8-bromoquinoline or 2-(2,2-dimethoxyethylthio)-8-bromoquinoline, with a yield of 42%. $^1$H NMR (400 MHz, $CDCl_3$) δ 7.90 (dd, *J*=7.6, 1.2 Hz, 1H), 7.81 (d, *J*=8.9 Hz, 1H), 7.59 (dd, *J*=7.9, 1.1 Hz, 1H), 7.13 (t, *J*=7.8 Hz, 1H), 6.76 (d, *J*=8.9 Hz, 1H), 6.10 (s, 1H), 4.23 (d, *J*=6.0 Hz, 2H), 2.85 (d, *J*=4.9 Hz, 3H). $^{13}$C NMR (125 MHz, $CDCl_3$) δ 171.5, 156.7, 144.4, 137.9, 133.1, 127.3, 124.8, 123.0, 121.2, 113.2, 46.8, 26.0. HRMS (ESI): Calculated for $C_{12}H_{13}BrN_3O$ [M+H]$^+$: 294.0237, Found: 294.0245.

Example 27 Preparation of Compound **2-27**

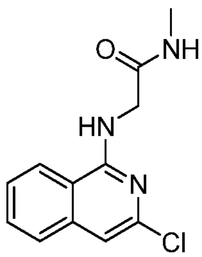

2-27

Compound 2-27 was obtained by the same preparation method in example 1 except that 2-(2,2-dimethoxyethoxy)quinoline or 2-(2,2-dimethoxyethylthio)quinoline was replaced with 3-chloro-2-(2,2-dimethoxyethoxy)isoquinoline or 3-chloro-2-(2,2-dimethoxyethylthio)isoquinoline, with a yield of 52%. $^1$H NMR (400 MHz, $CDCl_3$) δ 7.86 (d, *J*=8.4 Hz, 1H), 7.65-7.58 (m, 2H), 7.50-7.42 (m, 1H), 7.01 (s, 1H), 6.49 (s, 1H), 6.39 (s, 1H), 4.29 (d, *J*=5.0 Hz, 2H), 2.91 (d, *J*=4.8 Hz, 3H). $^{13}$C NMR (125 MHz, $CDCl_3$) δ 170.5, 154.7, 143.8, 138.7, 130.8, 126.4, 126.2, 122.0, 116.6, 109.4, 45.4, 26.3. HRMS (ESI) Calculated for $C_{12}H_{13}ClN_3O$ [M+H]$^+$: 250.0742, Found: 250.0739.

Example 28 Preparation of Compound **2-28**

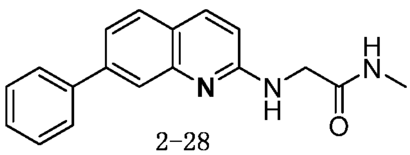

2-28

Compound 2-28 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-7-phenylquinoline or 2-(2,2-dimethoxyethylthio)-7-phenylquinoline, with a yield of 75%. $^1$H NMR (400 MHz, $CDCl_3$) δ 7.90 (d, *J*=8.8 Hz, 1H), 7.82 (t, 2H), 7.76 (d, *J*=9.1 Hz, 1H), 7.67 (d, *J*=8.0 Hz, 2H), 7.47 (t, *J*=7.6 Hz, 2H), 7.36 (t, *J*=7.4 Hz, 1H), 6.83 (s, 1H), 6.72 (d, *J*=8.8 Hz, 1H), 5.50 (s, 1H), 4.23 (d, *J*=5.1 Hz, 2H), 2.84 (d, *J*=4.9 Hz, 2H). $^{13}$C NMR (125 MHz, $CDCl_3$) δ 171.2, 156.0, 146.7, 140.7, 138.0, 135.7, 129.3, 128.9, 127.1, 127.1, 126.5, 125.4, 123.9, 112.3, 45.9, 26.2. HRMS (ESI) : Calculated for $C_{18}H_{18}N_3O$ [M+H]$^+$: 292.1444, Found: 292.1449.

Example 29 Preparation of Compound **2-29**

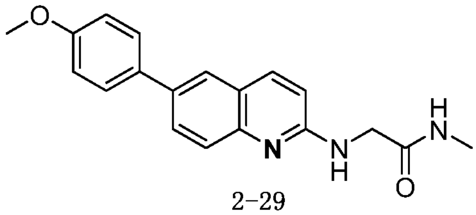

2-29

Compound 2-29 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-6-(4-methoxyphenyl)quinoline or 2-(2,2-dimethoxyethylthio)-6-(4-methoxyphenyl)quinoline, with a yield of 54%. $^1$H NMR (400 MHz, DMSO-*d*$_6$) δ 7.91 (d, *J*=9.0 Hz, 1H), 7.87 (d, *J*=1.9 Hz, 1H), 7.75 (dd, *J*=8.7, 2.0 Hz, 1H), 7.65 (d, *J*=8.7 Hz, 2H), 7.51 (d, 1H), 7.28 (t, *J*=5.5 Hz, 1H), 7.01 (d, *J*=8.7 Hz, 2H), 6.89 (d, *J*=8.9 Hz, 1H), 4.00 (d, *J*=5.5 Hz, 2H), 3.78 (s, 3H), 2.60 (d, *J*=4.0 Hz, 3H). $^{13}$C NMR (125 MHz, DMSO-*d*$_6$) δ 170.8, 159.0, 157.0, 147.1, 136.9, 133.3, 132.8, 128.1, 128.0, 126.6, 124.7, 123.8, 114.8, 114.1, 55.6, 44.5, 26.0. HRMS (ESI): Calculated for $C_{19}H_{20}N_3O_2$ $[M+H]^+$: 322.155, Found: 322.1549.

Example 30 Preparation of Compound **2-30**

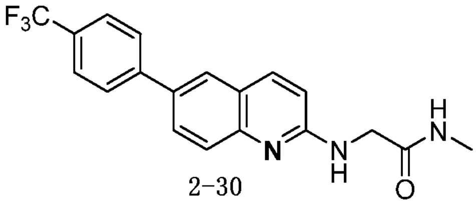

2-30

Compound 2-30 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-6-(4-trifluoromethylphenyl)quinoline or 2-(2,2-dimethoxyethylthio)-6-(4-trifluoromethylphenyl)quinoline, with a yield of 65%. $^1$H NMR (400 MHz, DMSO-*d*$_6$) δ 8.06 (d, *J*=1.8 Hz, 1H), 7.96 (t, 3H), 7.86 (dd, *J*=8.7, 2.0 Hz, 1H), 7.80 (d, *J*=8.3 Hz, 2H), 7.57 (d, 1H), 7.41 (t, *J*=5.8 Hz, 1H), 6.93 (d, *J*=8.9 Hz, 1H), 4.02 (d, *J*=5.4 Hz, 2H), 2.60 (d, *J*=3.9 Hz, 3H). $^{13}$C NMR (125 MHz, DMSO-*d*$_6$) δ 170.6, 157.5, 148.1, 144.4, 137.1, 131.7, 128.3, 127.5, 126.8, 126.3, 126.2, 126.2, 126.2, 123.8, 114.5, 44.4, 26.0. HRMS (ESI): Calculated for $C_{19}H_{17}FN_3O$ $[M+H]^+$: 360.1318, Found: 360.1317.

Example 31 Preparation of Compound **2-31**

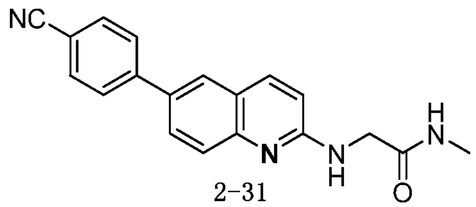

2-31

Compound 2-31 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-6-(4-cyanophenyl)quinoline or 2-(2,2-dimethoxyethylthio)-6-(4-cyanophenyl)quinoline, with a yield of 78%. $^1$H NMR (400 MHz, DMSO-*d*$_6$) δ 8.11 (s, 1H), 7.94 (dt, *J*=19.1, 9.6 Hz, 7H), 7.59 (d, *J*=8.8 Hz, 1H), 7.49 (t, *J*=5.6 Hz, 1H), 6.96 (d, *J*=9.1 Hz, 1H), 4.04 (d, *J*=5.2 Hz, 2H), 2.62 (d, *J*=4.4 Hz, 3H). $^{13}$C NMR (125 MHz, DMSO-*d*$_6$) δ 170.6, 157.6, 148.3, 144.9, 137.1, 133.3, 131.3, 128.2, 127.6, 126.9, 126.5, 123.7, 119.5, 114.5, 109.7, 44.4, 26.0. HRMS (ESI): Calculated for $C_{19}H_{17}N_4O$ $[M+H]^+$: 317.1397, Found: 317.1401.

Example 32 Preparation of Compound **2-32**

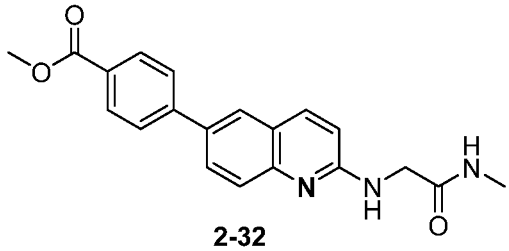

**2-32**

Compound 2-32 was obtained by the same preparation method in example 1 except that 2-(2,2-dimethoxyethoxy)quinoline or 2-(2,2-dimethoxyethylthio)quinoline was replaced with methyl 4-(2-(2,2-dimethoxyethoxy)quinolin-6-yl)benzoate or methyl 4-(2-(2,2-dimethoxyethylthio)quinolin-6-yl)benzoate, with a yield of 57%. $^1$H NMR (400 MHz, DMSO-$d_6$) δ 8.09 (d, *J*=2.0 Hz, 1H), 8.05 (d, *J*=8.4 Hz, 2H), 7.98 (d, *J*=8.9 Hz, 1H), 7.94-7.88 (m, 4H), 7.59 (d, *J*=8.7 Hz, 1H), 7.44 (t, *J*=5.5 Hz, 1H), 6.95 (d, *J*=8.9 Hz, 1H), 4.04 (d, *J*=5.6 Hz, 2H), 3.88 (s, 3H), 2.62 (d, *J*=4.6 Hz, 3H). $^{13}$C NMR (125 MHz, DMSO-$d_6$) δ 170.7, 166.6, 157.5, 148.1, 145.0, 137.1, 131.9, 130.3, 128.3, 128.2, 127.0, 126.8, 126.3, 123.8, 114.4, 56.5, 52.6, 26.0. HRMS (ESI): Calculated for $C_{20}H_{20}N_3O_3$ [M+H]$^+$: 350.1499, Found: 350.1501.

Example 33 Preparation of Compound **2-33**

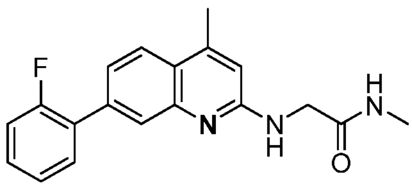

**2-33**

Compound 2-33 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-7-(2-fluorophenyl)-4-methylquinoline or 2-(2,2-dimethoxyethylthio)-7-(2-fluorophenyl)-4-methylquinoline, with a yield of 70%. $^1$H NMR (400 MHz, $CDCl_3$) δ 7.86 (d, *J*=8.9 Hz, 1H), 7.68 (d, *J*=8.4 Hz, 1H), 7.62 (d, *J*=7.9 Hz, 1H), 7.59-7.53 (m, 1H), 7.28-7.25 (m, 1H), 6.77 (d, *J*=8.9 Hz, 1H), 5.87 (s, 1H), 4.97 (s, 1H), 4.26 (dd, *J*=10.9, 2.6 Hz, 1H), 3.98 (dd, *J*=10.9, 6.2 Hz, 1H), 3.85 (s, 3H). $^{13}$C NMR (125 MHz, $CDCl_3$) δ 171.8, 155.6, 146.2, 138.2, 130.0, 127.5, 125.6, 123.5, 123.0, 112.5, 65.5, 58.0, 52.9. HRMS (ESI): Calculated for $C_{13}H_{15}N_2O_3$ [M+H]$^+$: 247.1077, Found: 247.1081.

**Preparation examples; reactions between a zolinium and various complicated molecules containing an amino acid residue:**

The present method is useful in chemical modifications of various complicated molecules containing an amino acid residue with good selectivity, as illustrated by the following preparation examples in which various complicated molecules containing an amino acid residue were reacted with a zolinium.

Example 34 Preparation of Compound **2-34**

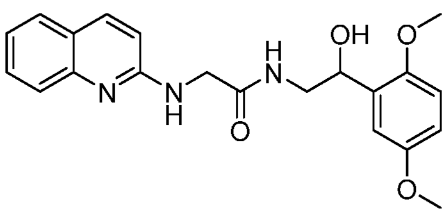

**2-34**

Compound 2-34 was obtained by the same preparation method in example 1 except that the nucleophile was replaced with 2-amino-N-(2-(2,5-dimethoxyphenyl)-2-hydroxyethyl)acetamide hydrochloride, with a yield of 72%. $^1$H NMR (500 MHz, $MeOD_4$) δ 7.88 (d, *J*=8.9 Hz, 1H), 7.62 (t, *J*=8.0 Hz, 2H), 7.53-7.48 (m, 1H), 7.24 (t, 1H), 7.00 (d, 1H), 6.81 (d, 1H), 6.75 (d, 1H), 6.70 (dd, *J*=8.9, 3.1 Hz, 1H), 5.08 (dd, *J*=7.1, 4.3 Hz, 1H), 4.12 (s, 2H), 3.72 (s, 3H), 3.68 (s, 3H), 3.56 (dd, 1H), 3.39 (dd, 1H). $^{13}$C NMR (126 MHz, MeOD4) δ 172.5, 156.8, 153.8, 150.3, 147.3, 137.2, 131.1, 129.1,

127.2, 125.4, 123.7, 122.0, 112.5, 112.3, 111.1, 66.7, 54.9, 54.6, 45.1, 44.6. HRMS (ESI): Calculated for $C_{21}H_{24}N_3O_4$ $[M+H]^+$: 382.1761, Found: 382.1761.

Example 35 Preparation of Compound **2-35**

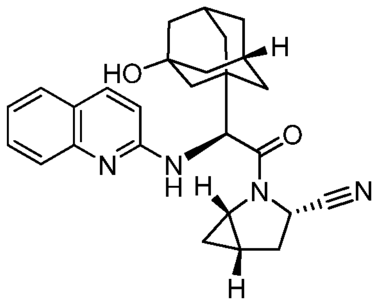

**2-35**

Compound 2-35 was obtained by the same preparation method in example 1 except that the nucleophile was replaced with saxagliptin hydrochloride, with a yield of 46%. $^1$H NMR (400 MHz, $CDCl_3$) δ 7.58-7.46 (m, 5H), 7.22 (t, *J*=7.3 Hz, 1H), 6.65 (d, *J*=8.7 Hz, 1H), 5.94 (d, *J*=9.5 Hz, 1H), 5.27 (d, *J*=9.4 Hz, 1H), 5.00 (dd, *J*=10.6, 2.4 Hz, 1H), 4.63 (td, *J*=6.2, 2.6 Hz, 1H), 2.75 (s, 1H), 2.51 (ddd, *J*=13.8, 10.7, 5.8 Hz, 1H), 2.36 (dd, *J*=13.7, 2.4 Hz, 1H), 2.32-2.23 (m, 3H), 2.07 (d, *J*=11.6 Hz, 1H), 2.00-1.92 (m, 2H), 1.86 (dd, *J*=25.7, 12.0 Hz, 2H), 1.73 (q, *J*=14.1 Hz, 6H), 1.59 (d, *J*=14.3 Hz, 3H), 1.28 (s, 2H), 1.18 (s, 1H), 1.17-1.11 (m, 1H). $^{13}$C NMR (126 MHz, $CDCl_3$) δ 171.7, 156.6, 147.5, 136.9, 129.2, 127.5, 126.1, 123.8, 122.2, 119.5, 113.2, 68.7, 58.9, 46.2, 45.3, 44.6, 44.2, 40.7, 38.3, 38.0, 37.7, 35.5, 30.6, 30.4, 30.3, 17.5, 13.7. HRMS (ESI): Calculated for $C_{27}H_{31}N_4O_2$ $[M+H]^+$: 443.2432, Found: 443.2442.

Example 36 Preparation of Compound **2-36**

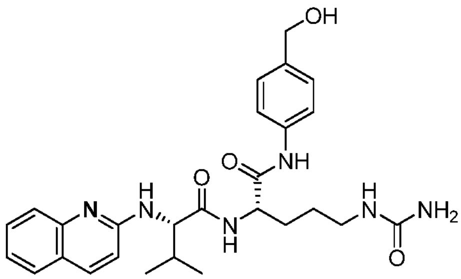

**2-36**

Compound 2-36 was obtained by the same preparation method in example 1 except that the nucleophile was replaced with (S)-2-((S)-2-amino-3-methylbutamido)-N-(4-(hydroxymethyl)phenyl)-5-ureidopentanamide hydrochloride, with a yield of 56%. $^1$H NMR (400 MHz, MeOD4) δ 7.87 (d, *J*=8.9 Hz, 1H), 7.63-7.58 (m, 2H), 7.47 (ddd, *J*=8.4, 7.0, 1.4 Hz, 1H), 7.39 (d, *J*=8.5 Hz, 2H), 7.25 (d, *J*=8.6 Hz, 2H), 7.22-7.17 (m, 1H), 6.91 (d, *J*=8.9 Hz, 1H), 4.58-4.53 (m, 3H), 4.49 (d, *J*=7.5 Hz, 1H), 3.37 (s, 2H), 3.09 (dt, *J*=13.3, 6.7 Hz, 1H), 2.99 (dt, *J*=13.5, 6.7 Hz, 1H), 2.30-2.19 (m, 1H), 1.94-1.84 (m, 1H), 1.80-1.68 (m, 1H), 1.60-1.43 (m, 2H), 1.11 (d, *J*=6.9 Hz, 3H), 1.09 (d, *J*=6.8 Hz, 3H). $^{13}$C NMR (126 MHz, MeOD4) δ 174.4, 170.9, 160.8, 157.0, 147.4, 137.4, 137.0, 136.9, 129.0, 127.1, 127.1, 125.4, 123.6, 121.9, 120.0, 112.6, 63.4, 60.9, 53.4, 48.4, 30.4, 29.3, 26.3, 18.7, 17.9. HRMS (ESI): Calculated for $C_{27}H_{35}N_6O_4$ $[M+H]^+$: 507.2724, Found: 507.2714.

Example 37 Preparation of Compound 2-37

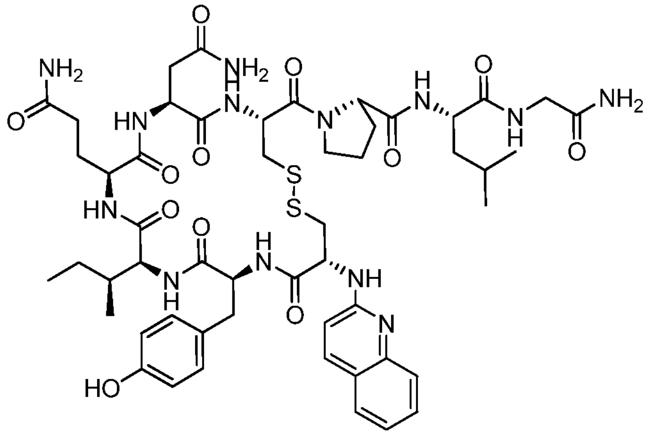

**2-37**

Compound 2-37 was obtained by the same preparation method in example 1 except that the nucleophile was replaced with oxytocin, with a yield of 42%. $^1$H NMR (400 MHz, MeOD4-$d_4$) δ 8.36 (d, *J*=9.2 Hz, 1H), 7.96 (d, *J*=7.8 Hz, 1H), 7.89 (t, *J*=7.8 Hz, 1H), 7.74 (d, *J*=8.3 Hz, 1H), 7.61 (t, *J*=7.4 Hz, 1H), 7.00 (d, 4H), 6.18 (s, 1H), 5.17 (s, 1H), 4.87 (d, *J*=6.8 Hz, 2H), 4.50-4.42 (m, 1H), 4.31 (dd, *J*=9.4, 5.4 Hz, 1H), 4.07 (d, *J*=6.5 Hz, 1H), 3.95 (d, *J*=7.6 Hz, 1H), 3.91 (d, 7=17.1 Hz, 1H), 3.81 (d, *J*=8.4 Hz, 1H), 3.76 (d, *J*=17.0 Hz, 1H), 3.68 (dt, *J*=10.4, 5.7 Hz, 1H), 3.42 (dt, *J*=13.6, 4.3 Hz, 2H), 3.37 (s, 4H), 2.91 (s, 1H), 2.71 (s, 1H), 2.39 (t, *J*=6.9 Hz, 2H), 2.31-2.14 (m, 3H), 2.03-1.83 (m, 4H), 1.68 (qd, *J*=9.7, 9.3, 5.1 Hz, 4H), 1.37-1.24 (m, 1H), 1.05 (d, *J*=6.7 Hz, 3H), 1.00 (t, *J*=7.4 Hz, 3H), 0.94 (d, *J*=6.3 Hz, 3H), 0.91 (d, *J*=6.1 Hz, 3H). $^{13}$C NMR (125 MHz, MeOD4) δ 176.6, 173.7, 173.5, 173.2, 172.9, 172.1, 168.4, 167.7, 157.7, 157.4, 155.6, 133.1, 129.8, 128.7, 127.2, 125.9, 121.8, 117.7, 115.8, 114.4, 113.5, 61.0, 60.8, 55.8, 54.9, 53.6, 53.0, 52.3, 50.6, 48.5, 48.2, 41.9, 39.7, 37.7, 35.7, 31.4, 29.0, 25.6, 24.5, 24.5, 22.1, 20.5, 14.6, 10.2. HRMS (ESI): Calculated for $C_{52}H_{70}N_{13}O_{12}S_2$ $[M+H]^+$: 1132.4742, Found: 1132.4742.

Example 38 Preparation of Compound 2-38

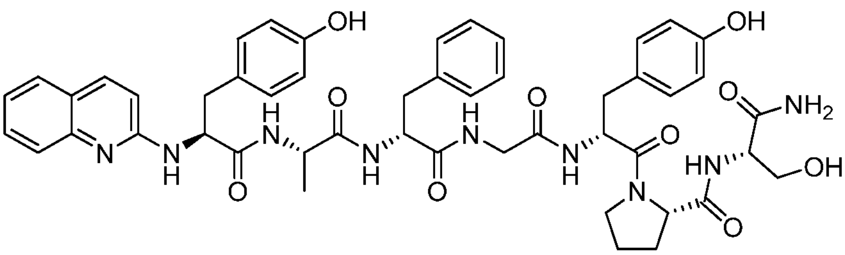

**2-38**

Compound 2-38 was obtained by the same preparation method in example 1 except that the nucleophile was replaced with dermorphin, with a yield of 47%. $^1$H NMR (400 MHz, DMSO-$d_6$) δ 9.25 (s, 1H), 9.17 (s, 1H), 8.27 (d, *J*=8.0 Hz, 1H), 8.20 (s, 1H), 8.12 (d, *J*=8.6 Hz, 1H), 8.00 (d, *J*=6.9 Hz, 1H), 7.82 (d, *J*=8.9 Hz, 1H), 7.75 (d, *J*=7.4 Hz, 1H), 7.59 (d, *J*=7.6 Hz, 1H), 7.49 (d, *J*=7.8 Hz, 1H), 7.46-7.38 (m, 1H), 7.18 (dd, *J*=13.4, 5.1 Hz, 5H), 7.12 (d, *J*=7.5 Hz, 3H), 7.10-7.05 (m, 3H), 6.85 (d, *J*=8.9 Hz, 1H), 6.66 (d, *J*=8.2 Hz, 2H), 6.62 (d, *J*=8.0 Hz, 2H), 4.91 (t, *J*=5.3 Hz, 1H), 4.74 (d, *J*=4.4 Hz, 1H), 4.60 (s, 1H), 4.47 (s, 1H), 4.35 (d, *J*=6.0 Hz, 1H), 4.26-4.04 (m, 2H), 3.78-3.69 (m, 1H), 3.68-3.54 (m, 4H), 2.97 (d, *J*=10.9 Hz, 3H), 2.87-2.76 (m, 1H), 2.69-2.60 (m, 1H), 2.57 (d, *J*=I2.8 Hz, 1H), 1.93 (d, *J*=51.9 Hz, 4H), 0.82 (d, *J*=6.9 Hz, 3H). $^{13}$C NMR (125 MHz, DMSO-$d_6$) δ 172.5, 172.3, 172.3, 171.6, 171.6, 171.0, 168.8, 156.6, 156.3, 156.1, 147.9, 138.2, 136.8, 130.7, 130.6, 129.6, 129.5, 128.8, 128.4, 128.1, 127.8, 126.6, 126.2, 123.5, 121.9, 115.5, 115.3, 113.6, 62.0, 60.4, 56.5, 55.5, 54.1, 53.1, 48.5, 47.4, 42.0, 38.1, 37.5, 36.7, 29.3, 24.9, 18.8. HRMS (ESI): Calculated for $C_{49}H_{54}N_9O_{10}$ $[M+H]^+$: 928.3999, Found: 928.3993.

Example 39 Preparation of Compound **2-39**

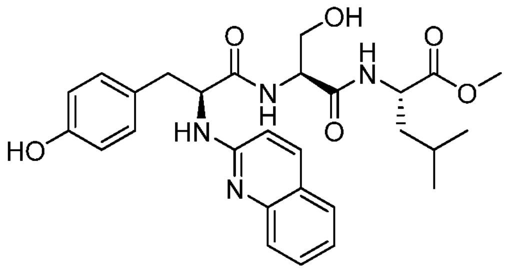

**2-39**

Compound 2-39 was obtained by the same preparation method in example 1 except that the nucleophile was replaced with tyroserleutide, with a yield of 56%. $^1$H NMR (400 MHz, DMSO-$d_6$) δ 9.16 (s, 1H), 8.20 (d, *J*=7.9 Hz, 1H), 7.98 (d, *J*=7.9 Hz, 1H), 7.84 (d, *J*=8.9 Hz, 1H), 7.60 (d, *J*=7.9 Hz, 1H), 7.50-7.42 (m, 2H), 7.21 (d, *J*=7.6 Hz, 1H), 7.17 (s, 1H), 7.13 (d, *J*=7.6 Hz, 2H), 6.85 (d, *J*=8.9 Hz, 1H), 6.62 (d, *J*=7.6 Hz, 2H), 4.84 (t, *J*=5.0 Hz, 1H), 4.76 (s, 1H), 4.31 (dd, *J*=14.0, 7.0 Hz, 2H), 4.15 (d, *J*=5.0 Hz, 1H), 3.61 (s, 3H), 3.19-3.17 (m, 3H), 1.58-1.44 (m, 2H), 1.41-1.35 (m, 2H), 0.81-0.75 (m, 6H). $^{13}$C NMR (125 MHz, DMSO-$d_6$) δ 173.1, 173.1, 170.5, 156.8, 156.1, 147.9, 136.8, 130.6, 129.4, 129.0, 127.9, 126.2, 123.5, 121.9, 115.3, 113.6, 62.1, 56.7, 55.3, 52.3, 50.6, 49.1, 37.2, 24.5, 23.1, 21.7. HRMS (ESI): Calculated for $C_{28}H_{35}N_4O_6$ [M+H]$^+$: 523.2551, Found: 523.2554.

Example 40 Preparation of Compound **2-40**

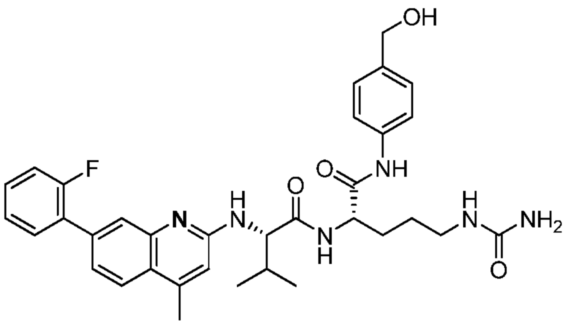

**2-40**

Compound 2-40 was obtained by the same preparation method in example 1 except that 2-(2,2-dimethoxyethoxy)quinoline or 2-(2,2-dimethoxyethylthio)quinoline was replaced with 2-(2,2-dimethoxyethoxy)-7-(2-fluorophenyl)-4-methylquinoline or 2-(2,2-dimethoxyethylthio)-7-(2-fluorophenyl)-4-methylquinoline, and the nucleophile was replaced with (S)-2-((S)-2-amino-3-methylbutamido)-N-(4-(hydroxymethyl)phenyl)-5-ureidopentanamide hydrochloride, with a yield of 52%. $^1$H NMR (500 MHz, MeOD-$d_4$) δ 7.88 (d, *J*=8.4 Hz, 1H), 7.80 (s, 1H), 7.51 (t, *J*=7.8 Hz, 1H), 7.44 (d, *J*=8.4 Hz, 1H), 7.42-7.37 (m, 1H), 7.33 (d, *J*=8.5 Hz, 2H), 7.28 (t, *J*=7.5 Hz, 1H), 7.24-7.21 (m, 1H), 7.20 (d, *J*=8.4 Hz, 2H), 6.82 (s, 1H), 4.56 (dd, *J*=9.0, 5.1 Hz, 1H), 4.52 (s, 2H), 4.48 (d, *J*=7.4 Hz, 1H), 3.11-3.04 (m, 1H), 3.01-2.94 (m, 1H), 2.61 (s, 3H), 2.31-2.22 (m, 1H), 1.89 (dt, *J*=14.3, 6.6 Hz, 1H), 1.78-1.68 (m, 1H), 1.57-1.47 (m, 2H), 1.13 (d, *J*=6.8 Hz, 3H), 1.11 (d, *J*=6.8 Hz, 3H). $^{13}$C NMR (125 MHz, MeOD4-$d_4$) δ 175.8, 172.3, 162.2, 160.3, 158.7, 148.8, 146.2, 138.8, 138.3, 137.9, 132.1, 130.5, 130.4, 128.4, 127.4, 125.7, 124.8, 124.6, 124.1, 121.5, 117.2, 117.0, 114.2, 64.8, 62.4, 54.8, 31.7, 30.7, 27.7, 20.1, 19.3, 18.7. HRMS (ESI): Calculated for $C_{34}H_{40}FN_6O_4$ [M+H]$^+$: 615.309, Found: 615.3081.

Example 41 Preparation of Compound **2-41**

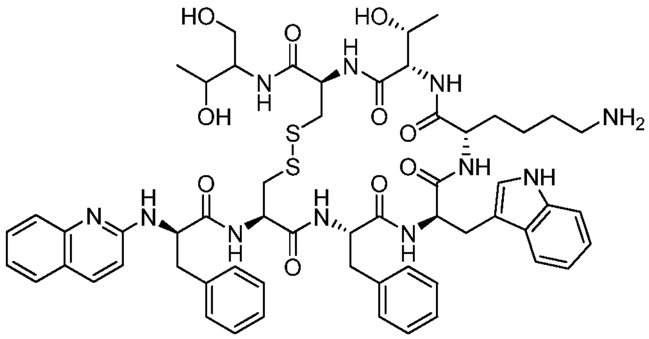

**2-41**

Compound 2-41 was obtained by the same preparation method in example 1 except that 2-(2,2-dimethoxyethoxy)quinoline was used and the nucleophile was replaced with octreotide acetate hydrochloride, with a yield of 41%. $^1$H NMR (500 MHz, Methanol-*d*$_4$) δ 8.27 (d, *J*=9.4 Hz, 1H), 7.85 (d, *J*=7.9 Hz, 1H), 7.78 (dd, *J*=14.4, 7.1 Hz, 2H), 7.53 (d, *J*=7.8 Hz, 1H), 7.48 (d, *J*=7.9 Hz, 1H), 7.40-7.36 (m, 3H), 7.27-7.22 (m, 3H), 7.19 (t, J=7.9 Hz, 4H), 7.16-7.10 (m, 3H), 7.04 (t, *J*=7.4 Hz, 1H), 7.00 (s, 1H), 5.25 (d, *J*=9.4 Hz, 2H), 4.68 (dd, *J*=8.8, 6.2 Hz, 1H), 4.49 (d, *J*=5.6 Hz, 1H), 4.40-4.31 (m, 1H), 4.23 (dd, *J*=11.0, 5.3 Hz, 1H), 4.17 (dd, *J*=6.4, 2.8 Hz, 1H), 4.02 (dd, *J*=10.9, 3.2 Hz, 1H), 3.89-3.85 (m, 1H), 3.74 (s, 2H), 3.50 (d, *J*=9.2 Hz, 1H), 3.33 (s, 2H), 3.18-2.90 (m, 8H), 2.86 (dd, J=13.8, 5.2 Hz, 1H), 2.61 (dq, *J*=18.7, 12.4, 9.4 Hz, 2H), 1.35-1.23 (m, 9H), 0.64-0.43 (m, 2H). HRMS (ESI): Calculated for $C_{58}H_{72}N_{11}O_{10}S_2$ [M+H]$^+$: 1146.49, Found: 1146.4891.

Example 42 Preparation of Compound **2-42**

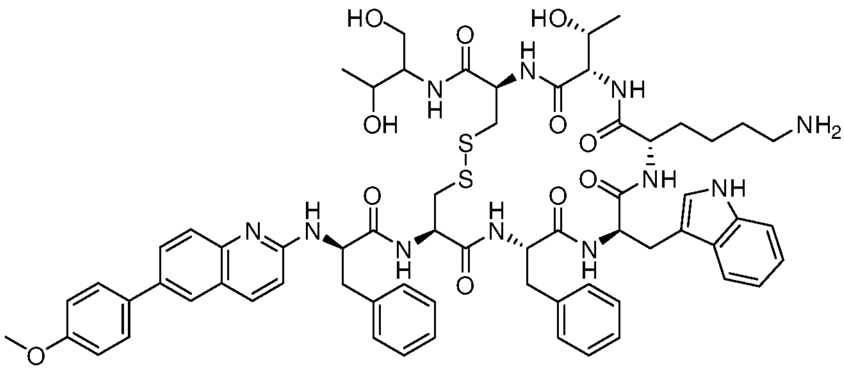

**2-42**

Compound 2-42 was obtained by the same preparation method in example 1 except that 2-(2,2-dimethoxyethoxy)-6-(4-methoxyphenyl)quinoline was used and the nucleophile was replaced with octreotide acetate hydrochloride, with a yield of 40%. $^1$H NMR (500 MHz, Methanol-*d*$_4$) δ 8.35 (d, *J*=9.4 Hz, 1H), 8.23 (d, *J*=8.7 Hz, 1H), 8.14-8.00 (m, 2H), 7.81 (d, *J*=9.0 Hz, 1H), 7.71-7.64 (m, 2H), 7.51 (d, *J*=8.0 Hz, 1H), 7.42 (d, *J*=7.6 Hz, 2H), 7.39 (d, *J*=8.1 Hz, 1H), 7.31 (t, *J*=7.5 Hz, 2H), 7.27-7.19 (m, 5H), 7.18-7.13 (m, 2H), 7.08 (dd, *J*=8.4, 6.7 Hz, 3H), 7.02 (s, 1H), 5.30 (t, *J*=l2.2 Hz, 2H), 4.71 (dd, *J*=8.9, 6.0 Hz, 1H), 4.54-4.47 (m, 1H), 4.44-4.33 (m, 1H), 4.25 (dd, *J*=10.9, 5.4 Hz, 1H), 4.23-4.16 (m, 1H), 4.06 (dd, *J*=11.0, 3.5 Hz, 1H), 3.89 (s, 4H), 3.77 (d, *J*=5.2 Hz, 2H), 3.54 (dd, *J*=14.0, 5.0 Hz, 1H), 3.39 (s, 2H), 3.23-2.94 (m, 8H), 2.89 (dd, *J*=13.8, 5.3 Hz, 1H), 2.65 (tt, *J*=20.1, 10.6 Hz, 2H), 1.28 (m, 9H), 0.69-0.50 (m, 2H). HRMS (ESI): Calculated for $C_{65}H_7gN_1O_{11}S_2$ [M+H]$^+$: 1252.5318, Found: 1252.5299.

Example 43 Preparation of Compound **2-43**

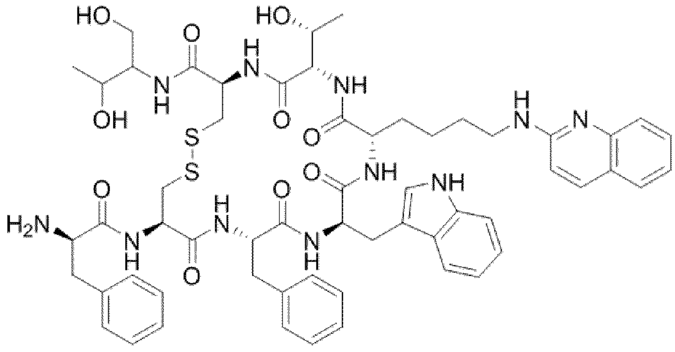

**2-43**

Compound 2-43 was obtained by the same preparation method in example 1 except that 2-(2,2-dimethoxyethylthio)quinoline or 2-(2,2-dimethoxyethylseleno)quinoline was used and the nucleophile was replaced with octreotide acetate hydrochloride, with a yield of 33%-41%. $^1$H NMR (500 MHz, Methanol-*d*$_4$) δ 7.84 (d, *J*=9.1 Hz, 1H), 7.68 (d, *J*=8.4 Hz, 1H), 7.61 (dd, *J*=8.0, 1.5 Hz, 1H), 7.53-7.49 (m, 1H), 7.48-7.42 (m, 1H), 7.36-7.24 (m, 11H), 7.20 (m, 1H), 7.05 (m, 2H), 6.95 (s, 1H), 6.74 (d, *J*=9.0 Hz, 1H), 5.19 (m, 1H), 5.13 (m, 1H), 4.69 (m, 1H), 4.47 (d, *J*=5.2 Hz, 1H), 4.43-4.34 (m, 1H), 4.17 (m, 1H), 4.08 (m, 1H), 4.01 (m, 1H), 3.86 (m, 1H), 3.82-3.76 (m, 1H), 3.73 (m, 1H), 3.67 (m, 1H), 3.32-3.25 (m, 2H), 3.16-3.09 (m, 2H), 3.07-2.85 (m, 7H), 2.83-2.76 (m, 1H), 1.78 (m, 1H), 1.42-1.31 (m, 3H), 1.28 (d, *J*=6.4 Hz, 3H), 1.21 (d, *J*=6.4 Hz, 3H), 0.80-0.60 (m, 2H). $^{13}$C NMR (125 MHz, Methanol-*d*$_4$) δ 174.44, 174.20, 173.98, 171.47, 171.41, 169.10, 157.46, 147.27, 137.07, 136.87, 136.66, 136.57, 129.24, 129.15, 129.08, 128.33, 128.08, 127.32, 127.04, 126.64, 126.52, 124.11, 123.13, 121.56, 121.16, 118.53, 117.82, 112.71, 111.12, 108.59, 67.02, 65.79, 61.31, 59.77, 56.37, 56.27, 55.80, 54.97, 54.26, 52.64, 52.46, 44.38 , 42.03, 40.71, 40.43, 39.29, 30.40, 28.48, 25.87, 22.70, 19.02, 18.81. HRMS (ESI): Calculated for $C_{58}H_{72}N_{11}O_{10}S_2$ [M+H]$^+$: 1146.4900, Found: 1146.4894.

Example 44 Preparation of Compound **2-44**

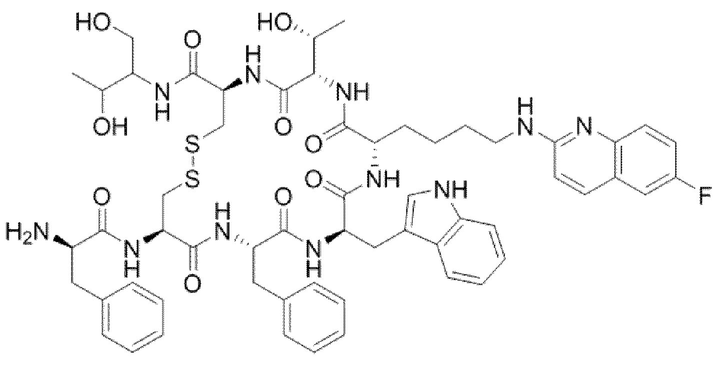

**2-44**

Compound 2-44 was obtained by the same preparation method in example 1 except that 6-fluoro-2-(2,2-dimethoxyethylthio)quinoline or 6-fluoro-2-(2,2-dimethoxyethylseleno)quinoline was used and the nucleophile was replaced with octreotide acetate hydrochloride, with a yield of 47%-52%. $^1$H NMR (600 MHz, Methanol-*d*$_4$) δ 7.79 (d, *J*=9.0 Hz, 1H), 7.68-7.62 (m, 1H), 7.42 (dd, *J*=7.4, 1.3 Hz, 1H), 7.34-7.26 (m, 11H), 7.22 (dd, *J*=8.0, 1.6 Hz, 2H), 7.02 (m, 2H), 6.92 (s, 1H), 6.75 (d, *J*=9.0 Hz, 1H), 5.14 (m, 2H), 4.65 (m, 1H), 4.44 (d, *J*=5.3 Hz, 1H), 4.41-4.32 (m, 1H), 4.14 (m, 1H), 4.09-4.05 (m, 1H), 4.02-3.93 (m, 2H), 3.81 (m, 1H), 3.72-3.61 (m, 2H), 3.26 (m, 2H), 3.16 (m, 1H), 3.09 (m, 1H), 3.03-2.85 (m, 7H), 2.77 (m, 1H), 1.75 (m, 1H), 1.43-1.30 (m, 3H), 1.25 (d, *J*=6.4 Hz, 3H), 1.18 (d, *J*=6.5 Hz, 3H), 0.65 (m, 2H). $^{13}$C NMR (150 MHz, Methanol-*d*$_4$) δ 174.35, 174.07, 171.69, 171.36, 171.33, 171.22, 168.85, 158.44, 157.01, 156.86, 144.04, 136.54, 136.41, 136.17, 135.57, 129.09, 128.93, 128.41, 127.97, 126.94, 126.43, 126.02, 125.96, 123.22, 123.16, 123.02, 121.04, 118.40, 117.87, 117.70, 113.76, 110.99, 110.74, 110.60, 108.46, 66.89, 65.68, 61.12, 59.63, 56.17, 55.05, 54.86, 54.11, 52.73, 52.29, 44.21, 42.02, 40.52, 39.16, 39.09, 30.25, 28.31, 25.72, 22.58, 18.86, 18.68. HRMS (ESI): Calculated for $C_{58}H_{71}FN_{11}O_{10}S_2$ [M+H]$^+$: 1164.4805, Found: 1164.482.

Example 45 Preparation of Compound **2-45**

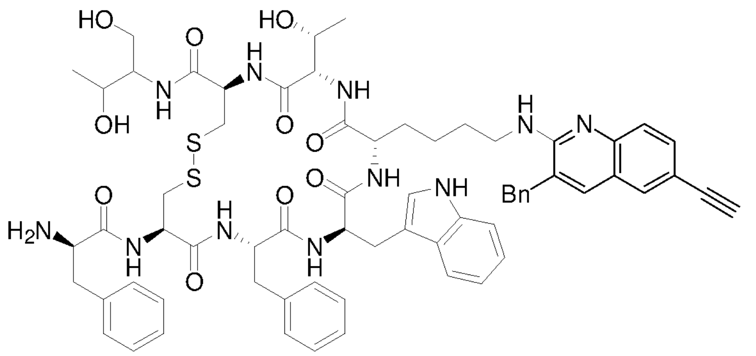

**2-45**

Compound 2-45 was obtained by the same preparation method in example 1 except that 6-alkynyl-3-benzyl-2-(2,2-dimethoxyethylthio)quinoline or 6-alkynyl-3-benzyl-2-(2,2-dimethoxyethylseleno)quinoline was used and the nucleophile was replaced with octreotide acetate hydrochloride, with a yield of 40%. $^1H$ NMR (600 MHz, Methanol-*d*$_4$) δ 7.66 (d, *J*=1.9 Hz, 1H), 7.62 (d, *J*=8.6 Hz, 1H), 7.52-7.46 (m, 2H), 7.44-7.38 (m, 1H), 7.36-7.17 (m, 16H), 7.04-6.96 (m, 2H), 6.88 (s, 1H), 5.21-5.09 (m, 2H), 4.71-4.60 (m, 1H), 4.43 (d, *J*=5.4 Hz, 1H), 4.38-4.31 (m, 1H), 4.16-4.11 (m, 1H), 4.08-4.04 (m, 1H), 3.98-3.90 (m, 4H), 3.83-3.78 (m, 1H), 3.71-3.61 (m, 2H), 3.41 (s, 1H), 3.34 (t, *J*=3.7 Hz, 2H), 3.18-3.12 (m, 1H), 3.11-3.06 (m, 1H), 3.03-2.85 (m, 7H), 2.79-2.72 (m, 1H), 1.74-1.63 (m, 1H), 1.34-1.28 (m, 1H), 1.27-1.25 (m, 2H), 1.23 (d, *J*=6.4 Hz, 3H), 1.18 (d, *J*=6.5 Hz, 3H), 0.65-0.45 (m, 2H). HRMS (ESI): Calculated for $C_{67}H_{78}N_{11}O_{10}S_2$ $[M+H]^+$: 1260.5369, Found: 1260.5374.

Example 46 Preparation of Compound **2-46**

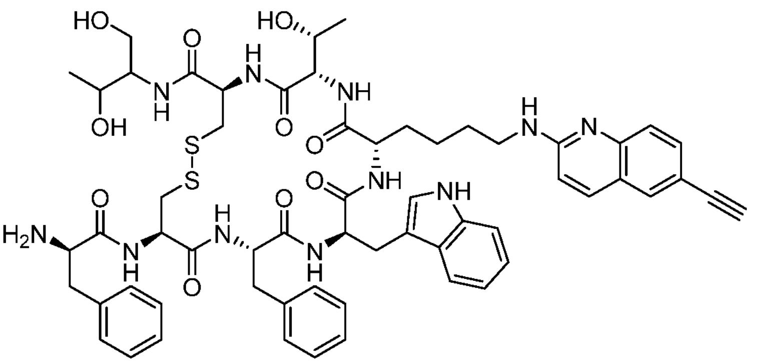

**2-46**

Compound 2-46 was obtained by the same preparation method in example 1 except that 6-alkynyl-2-(2,2-dimethoxyethylthio)quinoline or 6-alkynyl-2-(2,2-dimethoxyethylseleno)quinoline was used and the nucleophile was replaced with octreotide acetate hydrochloride, with a yield of 45%-78%. $^1H$ NMR (600 MHz, Methanol-*d*$_4$) δ 7.77 (d, *J*=9.0 Hz, 1H), 7.72 (d, *J*=1.9 Hz, 1H), 7.59 (d, *J*=8.6 Hz, 1H), 7.52 (dd, *J*=8.7, 1.9 Hz, 1H), 7.42 (dd, *J*=7.7, 1.3 Hz, 1H), 7.34-7.22 (m, 11H), 7.03 (m, 2H), 6.93 (s, 1H), 6.73 (d, *J*=9.0 Hz, 1H), 5.13 (m, 2H), 4.65 (m, 1H), 4.43 (d, *J*=5.3 Hz, 1H), 4.40-4.32 (m, 1H), 4.14 (m, 1H), 4.06 (m, 1H), 3.98 (m, 1H), 3.89 (t, *J*=7.1 Hz, 1H), 3.81 (m, 1H), 3.69 (m, 1H), 3.64 (m, 1H), 3.43 (s, 1H), 3.27 (m, 2H), 3.12 (m, 2H), 3.03-2.89 (m, 7H), 2.77 (m, 1H), 1.75 (m, 1H), 1.43-1.30 (m, 3H), 1.25 (d, *J*=6.4 Hz, 3H), 1.18 (d, *J*=6.5 Hz, 3H), 0.81-0.58 (m, 2H). $^{13}C$ NMR (125 MHz, Methanol-*d*$_4$) δ 174.02, 173.75, 172.10 , 171.02, 170.99 ,170.90, 168.55, 157.54, 147.13, 136.20, 136.08, 135.98, 135.65 , 131.67, 130.82, 128.71, 128.59, 128.00, 127.61, 126.58, 126.45, 126.07, 124.06, 122.67, 122.27, 120.70, 118.06, 117.35, 114.79, 110.64, 108.13, 82.82, 75.87, 66.51, 65.32, 60.79, 59.30, 55.84, 54.94, 54.52, 53.78, 52.31, 51.94, 43.87, 41.55 , 40.12, 39.17, 38.82, 29.89, 27.94, 25.37, 22.22, 18.52, 18.32. HRMS (ESI): Calculated for $C_{60}H_{72}N_{11}O_{10}S_2$ $[M+H]^+$: 1170.49, Found: 1170.4902.

Example 47 Preparation of Compound **2-47**

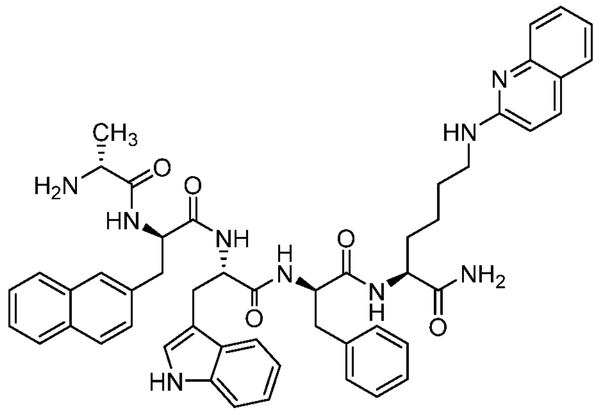

**2-47**

Compound 2-47 was obtained by the same preparation method in example 1 except that 2-(2,2-dimethoxyethylthio)quinoline or 2-(2,2-dimethoxyethylseleno)quinoline was used and the nucleophile was replaced with pralmorelin, with a yield of 50%-93%. $^1$H NMR (600 MHz, Methanol-d4) δ 7.72 (d, J=9.0 Hz, 1H), 7.69-7.65 (m, 1H), 7.60-7.51 (m, 3H), 7.49 (dd, J=8.0, 1.4 Hz, 1H), 7.43 (s, 1H), 7.42-7.37 (m, 1H), 7.35 (d, J=7.9 Hz, 1H), 7.33-7.28 (m, 2H), 7.23 (d, J=8.2 Hz, 1H), 7.16-6.99 (m, 8H), 6.96-6.89 (m, 1H), 6.81 (s, 1H), 6.65 (d, J=9.0 Hz, 1H), 4.54-4.50 (m, 1H), 4.49-4.44 (m, 1H), 4.31-4.26 (m, 1H), 4.03-3.98 (m, 1H), 3.52 (q, J=7.0 Hz, 1H), 3.33-3.21 (m, 2H), 3.04-2.97 (m, 1H), 2.97-2.92 (m, 1H), 2.90-2.85 (m, 1H), 2.84-2.77 (m, 2H), 2.66-2.61 (m, 1H), 1.74-1.63 (m,1H), 1.52-1.37 (m, 3H), 1.18 (d, *J*=7.1 Hz, 3H), 1.07-0.98 (m, 2H). $^{13}$C NMR (125 MHz, Methanol-*d*$_4$) δ 176.63, 175.74, 172.74, 172.29, 171.88, 157.58, 147.59, 136.83, 136.60, 134.18, 133.43, 132.46, 129.06, 128.95, 128.18, 128.13 , 127.62 , 127.58, 127.36, 127.21, 127.14, 127.06, 126.55, 125.64, 125.23, 124.43, 123.28, 123.15, 121.38, 121.10, 118.56, 117.82, 112.61, 111.05, 109.18, 55.66, 54.66, 54.39, 53.23, 49.88, 40.64, 37.28, 36.64, 30.89, 28.45, 27.01, 22.99, 19.63. HRMS (ESI): Calculated for $C_{51}H_{56}N_9O_5$ [M+H]$^+$: 874.4399, Found: 874.4393.

Example 48 Preparation of Compound **2-48**

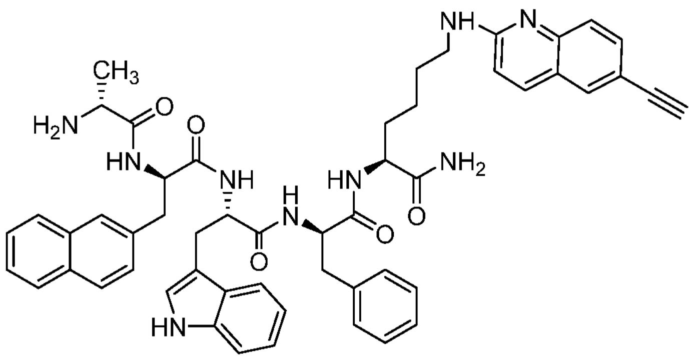

**2-48**

Compound 2-48 was obtained by the same preparation method in example 1 except that 6-alkynyl-2-(2,2-dimethoxyethylthio)quinoline or 6-alkynyl-2-(2,2-dimethoxyethylseleno)quinoline was used and the nucleophile was replaced with pralmorelin, with a yield of 48%-90%. $^1$H NMR (600 MHz, Methanol-*d*$_4$) δ 7.76 (dd, *J*=9.1, 3.6 Hz, 2H), 7.71 (d, *J*=1.9 Hz, 1H), 7.69-7.65 (m, 2H), 7.57-7.53 (m, 2H), 7.51 (dd, *J*=8.6, 1.9 Hz, 1H), 7.45 (m, 1H), 7.43-7.39 (m, 2H), 7.32 (m, 1H), 7.24-7.20 (m, 3H), 7.17-7.14 (m, 1H), 7.11 (m, 3H), 7.02 (m, 1H), 6.92 (s, 1H), 6.76 (d, *J*=9.0 Hz, 1H), 4.61 (dd, *J*=8.8, 5.9 Hz, 1H), 4.57 (dd, *J*=7.9, 6.4 Hz, 1H), 4.38 (dd, *J*=8.7, 7.0 Hz, 1H), 4.10 (m, 1H), 3.80-3.75 (m, 1H), 3.45 (s, 1H), 3.42-3.32 (m, 2H), 3.07 (m, 2H), 2.98 (m, 1H), 2.90 (m, 2H), 2.72 (m, 1H), 1.82-1.74 (m, 1H), 1.62-1.49 (m, 3H), 1.34 (d, *J*=7.0 Hz, 3H), 1.17-1.08 (m, 2H). $^{13}$C NMR (150 MHz, Methanol-d4) δ 176.09, 172.84, 172.53, 171.20, 170.31, 157.51, 146.48, 136.85, 136.58, 136.48, 134.21, 133.47, 132.50, 132.37, 131.33, 128.97, 128.20, 127.74, 127.56, 127.39, 127.21, 127.19, 126.96, 126.60, 125.75, 125.34, 123.93, 123.25, 122.56, 121.08, 118.53, 117.89, 115.62, 111.04, 109.34, 83.12, 76.61, 55.85, 55.09, 54.16, 53.33, 48.73, 40.70, 36.95, 36.49, 30.69, 28.20, 27.37, 23.01, 16.30 . HRMS (ESI): Calculated for $C_{53}H_{55}N_9NaO_5$ [M+Na]$^+$: 920.4218, Found: 920.4221.

Example 49 Preparation of Compound **2-49**

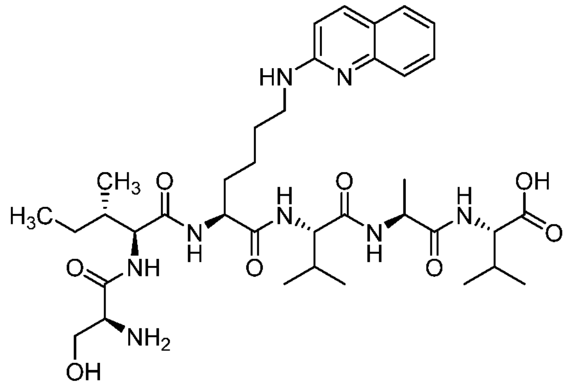

**2-49**

Compound 2-49 was obtained by the same preparation method in example 1 except that 2-(2,2-dimethoxyethylthio)quinoline or 2-(2,2-dimethoxyethylseleno)quinoline was used and the nucleophile was replaced with hexapeptide-10, with a yield of 40%-83%. $^1$H NMR (500 MHz, Methanol-$d_4$) δ 7.82 (d, *J*=9.0 Hz, 1H), 7.63 (d, *J*=8.4 Hz, 1H), 7.59 (dd, *J*=7.9, 1.5 Hz, 1H), 7.49 (m, 1H), 7.20-7.14 (m, 1H), 6.78 (d, *J*=9.0 Hz, 1H), 4.48 (d, *J*=7.1 Hz, 1H), 4.43 (dd, *J*=9.3, 5.3 Hz, 1H), 4.31 (d, *J*=6.3 Hz, 1H), 4.26 (d, *J*=7.0 Hz, 1H), 4.18 (d, *J*=5.1 Hz, 1H), 3.74 (m, 1H), 3.66 (m, 1H), 3.55 (m, 1H), 3.46 (m, 2H), 2.16 (m, 2H), 1.97-1.84 (m, 2H), 1.80-1.65 (m, 3H), 1.55 (m, 3H), 1.38 (d, *J*=7.1 Hz, 3H), 1.23 (m, 1H), 1.01-0.88 (m, 18H). $^{13}$C NMR (125 MHz, Methanol-$d_4$) δ 176.80, 174.98, 172.87, 172.40, 171.65, 157.62, 147.62, 136.81, 129.01, 127.21, 124.45, 123.16, 121.34, 112.72, 64.50, 60.12, 58.63, 58.29, 55.93, 53.59, 49.22, 40.66, 39.06, 36.62, 31.29, 30.61, 28.49, 24.58, 23.24, 18.82, 18.55, 17.32, 17.10, 16.67, 14.70, 10.36. HRMS (ESI): Calculated for $C_{37}H_{59}N_8O_8$ $[M+H]^+$: 743.445, Found: 743.4437.

Example 50 Preparation of Compound **2-50**

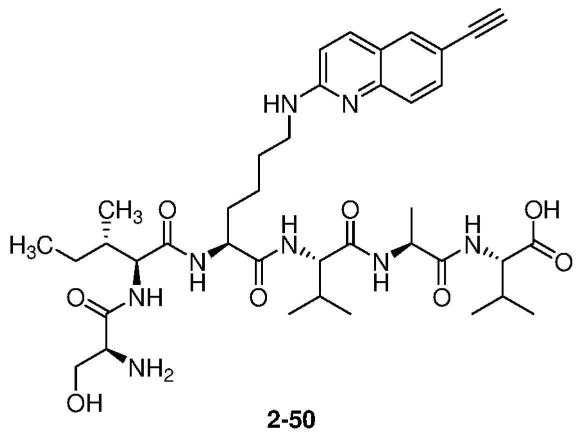

**2-50**

Compound 2-50 was obtained by the same preparation method in example 1 except that 6-alkynyl-2-(2,2-dimethoxyethylthio)quinoline or 6-alkynyl-2-(2,2-dimethoxyethylseleno)quinoline was used and the nucleophile was replaced with hexapeptide-10, with a yield of 40%-90%. $^1$H NMR (600 MHz, Methanol-$d_4$) δ 7.79-7.76 (m, 1H), 7.71 (d, *J*=1.8 Hz, 1H), 7.54 (d, *J*=8.6 Hz, 1H), 7.50 (dd, *J*=8.6, 1.9 Hz, 1H), 6.78 (d, *J*=9.0 Hz, 1H), 4.49-4.39 (m, 2H), 4.28 (d, *J*=6.7 Hz, 1H), 4.21 (d, *J*=7.0 Hz, 1H), 4.18 (d, *J*=4.9 Hz, 1H), 3.72-3.67 (m, 1H), 3.59 (s, 1H), 3.55-3.54 (m, 1H), 3.47-3.43 (m, 2H), 3.43-3.42 (m, 1H), 2.17-2.09 (m, 2H), 1.86 (m, 2H), 1.69 (m, 3H), 1.55-1.50 (m, 2H), 1.47 (d, *J*=9.1 Hz, 1H), 1.34 (d, *J*=7.1 Hz, 3H), 1.22-1.16 (m, 1H), 0.96-0.90 (m, 18H). HRMS (ESI): Calculated for $C_{39}H_{59}N_8O_8$ $[M+H]^+$: 767.445, Found: 767.4458.

Example 51 Preparation of Compound **2-51**

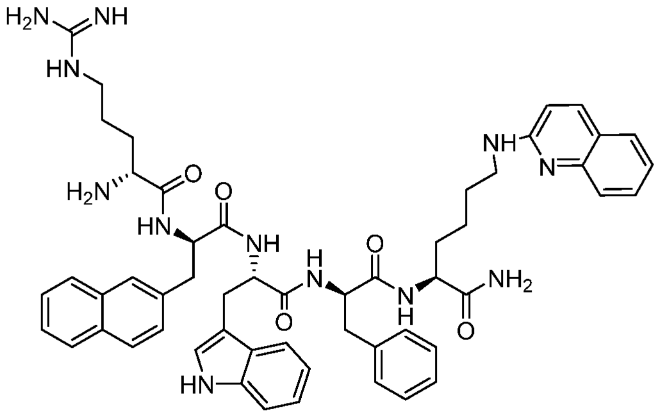

**2-51**

Compound 2-51 was obtained by the same preparation method in example 1 except that 2-(2,2-dimethoxyethylthio)quinoline or 2-(2,2-dimethoxyethylseleno)quinoline was used and the nucleophile was replaced with arginine-pralmorelin, with a yield of 45%-57%. $^1$H NMR (600 MHz, Methanol-*d*$_4$) δ 7.83 (d, *J*=9.0 Hz, 1H), 7.77 (dd, *J*=7.5, 1.9 Hz, 1H), 7.71 (d, *J*=8.5 Hz, 2H), 7.67-7.63 (m, 1H), 7.62-7.58 (m, 2H), 7.50 (m, 1H), 7.45-7.39 (m, 3H), 7.35-7.31 (m, 1H), 7.28 (dd, *J*=8.5, 1.7 Hz, 1H), 7.24-7.18 (m, 3H), 7.15 (d, *J*=7.4 Hz, 1H), 7.12-7.08 (m, 3H), 7.03 (m, 1H), 6.89 (s, 1H), 6.77 (d, *J*=9.0 Hz, 1H), 4.70 (m, 1H), 4.55 (m, 1H), 4.34 (m, 1H), 4.03 (m, 1H), 3.57 (m, 1H),3.36 (qm, *J*=7.3 Hz, 2H), 3.14-2.94 (m, 5H), 2.92-2.81 (m, 2H), 2.64 (m, 1H), 1.85-1.74 (m, 1H), 1.64 (m, 2H), 1.54 (m, 3H), 1.47-1.35 (m, 2H), 1.17-1.05 (m, 2H). $^{13}$C NMR (125 MHz, Methanol-d$_4$) δ 175.71, 172.51, 172.38, 171.97, 171.05, 156.69, 156.45, 145.54, 137.12, 136.05, 135.85, 133.75, 132.97, 132.00, 129.05, 128.43, 127.72, 127.24, 127.06, 126.94, 126.73, 126.70, 126.50, 126.15, 125.27, 124.86, 122.89, 122.87, 122.40, 121.43, 120.58, 118.06, 117.41, 112.13, 110.55, 108.75, 55.55, 54.29, 53.66, 53.14, 52.52, 40.33, 40.01, 36.72, 35.93, 30.12, 29.30, 27.75, 27.06, 23.16, 22.62HRMS (ESI): Calculated for $C_{54}H_{63}N_{12}O_5$ [M+H]$^+$: 959.5039, Found: 959.5025.

Example 52 Preparation of Compound **2-52**

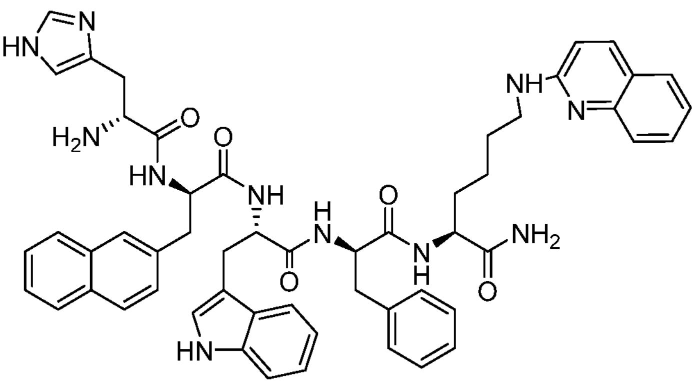

**2-52**

Compound 2-52 was obtained by the same preparation method in example 1 except that 2-(2,2-dimethoxyethylthio)quinoline or 2-(2,2-dimethoxyethylseleno)quinoline was used and the nucleophile was replaced with histidine-pralmorelin, with a yield of 42%-91%. $^1$H NMR (600 MHz, Methanol-*d*$_4$) δ 7.84 (d, *J*=9.0 Hz, 1H), 7.77-7.72 (m, 1H), 7.65 (dd, *J*=8.3, 3.0 Hz, 3H), 7.60 (dd, *J*=7.9, 1.4 Hz, 1H), 7.57 (s, 1H), 7.55-7.49 (m, 2H), 7.45 (d, *J*=7.9 Hz, 1H), 7.42-7.36 (m, 2H), 7.32 (d, *J*=8.1 Hz, 1H), 7.21 (m, 4H), 7.16-7.06 (m, 4H), 7.03 (m, 1H), 6.92 (s, 1H), 6.81 (s, 1H), 6.77 (d, *J*=9.0 Hz, 1H), 4.65 (dd, *J*=8.5, 6.0 Hz, 1H), 4.60-4.52 (m, 1H), 4.37 (m, 1H), 4.10 (m, 1H), 3.79 (m, 1H), 3.34 (m, 2H), 3.10 (m, 1H), 3.04 (m, 1H), 3.00-2.81 (m, 5H), 2.66 (m, 1H), 1.82-1.72 (m, 1H), 1.54 (m, 3H), 1.11 (m, 2H). $^{13}$C NMR (125 MHz, Methanol-*d*$_4$) δ 175.74, 175.43, 172.37, 172.17, 171.07, 170.56, 155.86, 144.10, 137.77, 136.08, 135.96, 134.90, 133.60, 132.95, 131.99, 131.33, 129.45, 128.46, 127.67, 127.21, 127.07, 127.04, 126.90, 126.73, 126.67, 126.48, 126.07, 125.22, 124.82, 122.79, 122.15, 121.96, 121.89, 120.60, 118.05, 117.40, 116.44, 112.15, 110.56, 108.90, 55.36, 54.39, 53.82, 52.90, 47.95, 40.47, 36.78, 35.93, 30.10, 29.28, 27.58, 26.88, 22.48.HRMS (ESI): Calculated for $C_{54}H_{58}N_{11}O_5$ [M+H]$^+$: 940.4617, Found: 940.4643.

Example 53 Preparation of Compound **2-53**

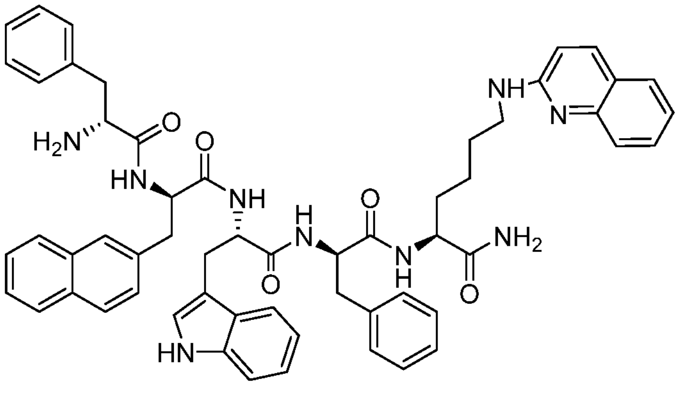

**2-53**

Compound 2-53 was obtained by the same preparation method in example 1 except that 2-(2,2-dimethoxyethylthio)quinoline or 2-(2,2-dimethoxyethylseleno)quinoline was used and the nucleophile was replaced with phenylalanine-pralmorelin, with a yield of 43%-47%. $^1$H NMR (600 MHz, Methanol-$d_4$) δ 7.79 (d, *J*=9.1 Hz, 1H), 7.72-7.67 (m, 1H), 7.61-7.52 (m, 4H), 7.45 (m, 2H), 7.37 (d, *J*=7.9 Hz, 1H), 7.35-7.31 (m, 2H), 7.25 (d, *J*=8.1 Hz, 1H), 7.16 (m, 6H), 7.11-7.05 (m, 4H), 7.03 (dd, *J*=7.6, 5.9 Hz, 3H), 6.96 (m, 1H), 6.81 (s, 1H), 6.70 (d, *J*=9.0 Hz, 1H), 4.59 (m, 1H), 4.47 m, 1H), 4.30 (dd, *J*=8.5, 7.0 Hz, 1H), 4.05 (m, 1H), 3.66 (dd, *J*=8.6, 4.9 Hz, 1H), 3.32-3.28 (m, 1H), 3.27-3.25 (m, 1H), 3.06-2.79 (m, 6H), 2.63 (m, 2H), 1.75-1.65 (m, 1H), 1.46 (m, 3H), 1.09-0.99 (m, 2H). $^{13}$C NMR (150 MHz, Methanol-$d_4$) δ 176.01, 172.86, 172.44, 171.20, 156.34, 138.20, 136.57, 136.50, 135.15, 134.05, 133.46, 132.51, 129.96, 129.07, 128.96, 128.54, 128.18, 127.74, 127.60, 127.41, 127.23, 127.18, 127.09, 127.00, 126.57, 125.75, 125.34, 123.28, 122.69, 122.48, 122.43, 121.08, 118.53, 117.87, 112.83, 111.05, 109.23, 55.86, 54.81, 54.69, 54.28, 53.17, 40.96, 38.35, 37.27, 36.32, 30.63, 27.99, 27.39, 22.91.HRMS (ESI): Calculated for $C_{57}H_{60}N_9O_5$ [M+H]$^+$: 950.4712, Found: 950.4708.

Example 54 Preparation of Compound **2-54**

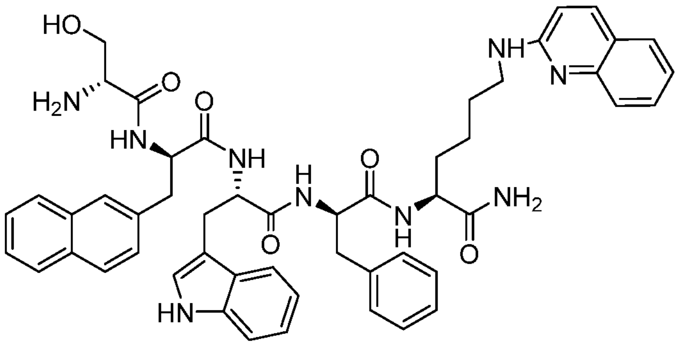

**2-54**

Compound 2-54 was obtained by the same preparation method in example 1 except that 2-(2,2-dimethoxyethylthio)quinoline or 2-(2,2-dimethoxyethylseleno)quinoline was used and the nucleophile was replaced with serine-pralmorelin, with a yield of 40%-86%. $^1$H NMR (600 MHz, Methanol-$d_4$) δ 7.82 (d, *J*=9.0 Hz, 1H), 7.75 (dd, *J*=6.1, 3.4 Hz, 1H), 7.66-7.58 (m, 4H), 7.53-7.49 (m, 2H), 7.46 (m, 1H), 7.40 (m, 2H), 7.33 (m, 1H), 7.23-7.10 (m, 6H), 7.09-7.05 (m, 2H), 7.04 (m, 1H), 6.94 (s, 1H), 6.75 (d, *J*=9.0 Hz, 1H), 4.66 (m, 1H), 4.51 (m, 1H), 4.41 (m, 1H), 4.14 (m, 1H), 3.68 (d, *J*=5.5 Hz, 2H), 3.57 (m, 1H), 3.36 (m, 2H), 3.15 (m, 1H), 3.07-3.00 (m, 1H), 2.99-2.84 (m, 3H), 2.74 (m, 1H), 1.79 (m, 1H), 1.63-1.50 (m, 3H), 1.16 (m, 2H). $^{13}$C NMR (125 MHz, Methanol-$d_4$) δ 175.84, 172.82, 172.31, 171.58, 170.76, 156.82, 137.66, 136.62, 136.53, 134.13, 133.46, 132.48, 129.59, 128.96, 128.17, 127.66, 127.51, 127.44, 127.34, 127.23, 127.14, 126.98, 126.54, 125.66, 125.27, 123.26, 122.87, 122.01, 121.12, 118.54, 117.87, 112.71, 111.07, 109.30, 56.92, 55.53, 55.36, 54.82, 54.57, 53.22, 40.84, 37.15, 36.49, 30.79, 28.22, 27.12, 22.95. HRMS (ESI): Calculated for $C_{51}H_{56}N_9O_6$ [M+H]$^+$: 890.4348, Found: 890.4356.

Example 55 Preparation of Compound **2-55**

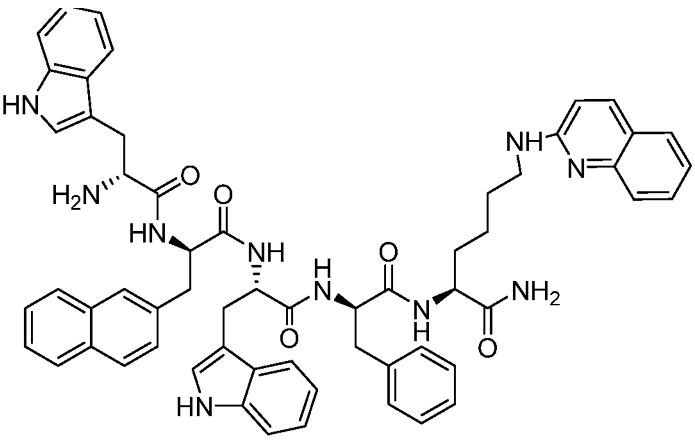

**2-55**

Compound 2-55 was obtained by the same preparation method in example 1 except that 2-(2,2-dimethoxyethylthio)quinoline or 2-(2,2-dimethoxyethylseleno)quinoline was used and the nucleophile was replaced with tryptophan-pralmorelin, with a yield of 41%-98%. $^1H$ NMR (600 MHz, Methanol-$d_4$) δ 7.81 (d, $J$=9.1 Hz, 1H), 7.73 (dd, $J$=6.2, 3.4 Hz, 1H), 7.65-7.61 (m, 1H), 7.61-7.57 (m, 2H), 7.57-7.53 (m, 2H), 7.50 (m, 1H), 7.45 (m, 1H), 7.38 (m, 3H), 7.33 (m, 2H), 7.24-7.17 (m, 3H), 7.16-7.02 (m, 8H), 6.99 (m, 1H), 6.90 (s, 1H), 6.73 (d, $J$=9.0 Hz, 1H), 4.66 (dd, $J$=7.8, 6.3 Hz, 1H), 4.53 (dd, $J$=7.8, 6.4 Hz, 1H), 4.38 (dd, $J$=8.5, 7.0 Hz, 1H), 4.12 (m, 1H), 3.82 (dd, $J$=7.6, 5.3 Hz, 1H), 3.36-3.31 (m, 1H), 3.28 (m, 1H), 3.17 (m, 1H), 3.05-2.97 (m, 3H), 2.96-2.88 (m, 3H), 2.69 (m, 1H), 1.80-1.71 (m, 1H), 1.50 (m, 3H), 1.15-1.02 (m, 2H). $^{13}C$ NMR (125 MHz, Methanol-$d_4$) δ 175.85, 172.90, 172.51, 171.38, 156.89, 145.98, 137.57, 136.75, 136.60, 136.51, 133.95, 133.41, 132.46, 129.54, 128.95, 128.18, 127.64, 127.57, 127.42, 127.38, 127.25, 127.20, 127.13, 127.03, 126.56, 125.66, 125.27, 124.13, 123.38, 123.29, 122.90, 121.94, 121.30, 121.11, 118.71, 118.56, 117.87, 117.84, 112.68, 111.14, 111.08, 109.28, 107.60, 55.78, 54.65, 54.40, 54.03, 53.27, 40.80, 37.35, 36.41, 30.68, 28.55, 28.16, 27.29, 22.95. HRMS (ESI): Calculated for $C_{59}H_{61}N_{10}O_5$ $[M+H]^+$: 989.4821, Found: 989.4829.

Example 56 Preparation of Compound **2-56**

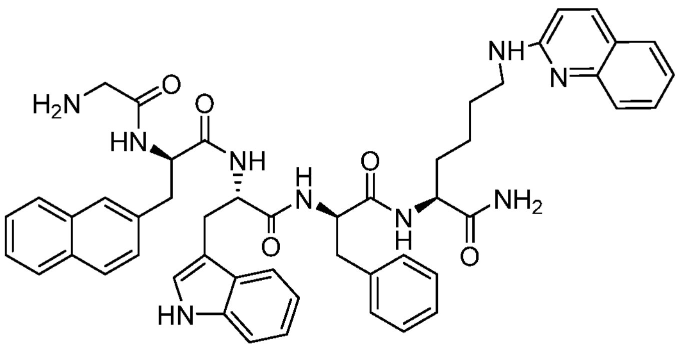

**2-56**

Compound 2-56 was obtained by the same preparation method in example 1 except that 2-(2,2-dimethoxyethylthio)quinoline or 2-(2,2-dimethoxyethylseleno)quinoline was used and the nucleophile was replaced with glycine-pralmorelin, with a yield of 35%-83%. $^1H$ NMR (600 MHz, Methanol-$d_4$) δ 7.96 (d, $J$=9.2 Hz, 1H), 7.78-7.75 (m, 1H), 7.72 (d, $J$=8.4 Hz, 1H), 7.70-7.65 (m, 3H), 7.60 (m, 1H), 7.54 (d, $J$=1.6 Hz, 1H), 7.45 (m, 1H), 7.43-7.39 (m, 2H), 7.34-7.31 (m, 2H), 7.24-7.20 (m, 3H), 7.15 (m, 1H), 7.11 (m, 3H), 7.03 (m, 1H), 6.91 (s, 1H), 6.87 (d, $J$=9.2 Hz, 1H), 4.68 (dd, $J$=8.5, 6.0 Hz, 1H), 4.55 (dd, $J$=7.8, 6.7 Hz, 1H), 4.38 (dd, $J$=8.5, 6.9 Hz, 1H), 4.13 (m, 1H), 3.60-3.48 (m, 2H), 3.39 (m, 2H), 3.06 (m, 2H), 2.92 (m, 3H), 2.71 (m, 1H), 1.85-1.76 (m, 1H), 1.58 (m, 3H), 1.19-1.11 (m, 2H). $^{13}C$ NMR (125 MHz, Methanol-$d_4$) δ 175.93, 172.88, 172.43, 171.37, 166.52, 139.37, 138.65, 136.58, 136.53, 134.03, 133.47, 132.50, 130.68, 128.96, 128.17, 127.87, 127.70, 127.51, 127.37, 127.22, 127.16, 126.95, 126.53, 125.73, 125.34, 123.26, 122.30, 121.09, 118.52, 117.86, 113.04, 111.06, 109.26, 55.77, 54.85, 54.35, 53.15, 41.27, 40.21, 37.37, 36.40, 30.54, 27.73, 27.29, 22.83. HRMS (ESI): Calculated for $C_{50}H_{53}N_9NaO_5$ $[M+Na]^+$: 882.4062, Found: 882.4056.

Example 57 Preparation of Compound **2-57**

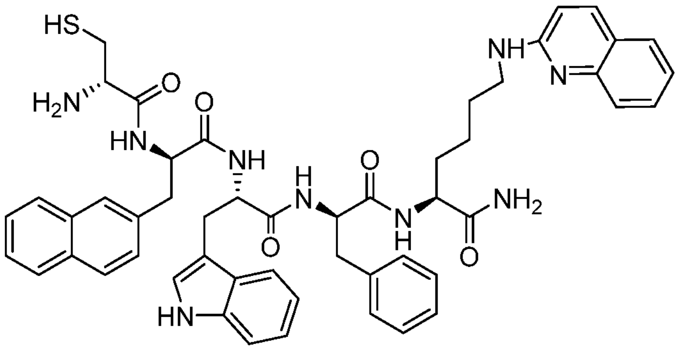

**2-57**

Compound 2-57 was obtained by the same preparation method in example 1 except that 2-(2,2-dimethoxyethylthio)quinoline or 2-(2,2-dimethoxyethylseleno)quinoline was used and the nucleophile was replaced with cysteine-pralmorelin, with a yield of 55%. $^1$H NMR (600 MHz, Methanol-$d_4$) δ 8.31-8.15 (m, 1H), 8.06 (d, $J$=9.4 Hz, 1H), 7.88 (d, $J$=8.5 Hz, 1H), 7.81-7.68 (m, 3H), 7.68-7.62 (m, 2H), 7.56 (s, 1H), 7.49-7.42 (m, 2H), 7.39 (m, 2H), 7.30 (d, $J$=8.2 Hz, 1H), 7.22 (m, 3H), 7.14 (d, $J$=7.8 Hz, 3H), 7.07 (t, $J$=7.6 Hz, 1H), 7.00 (d, $J$=7.4 Hz, 1H), 6.94 (s, 1H), 4.64 (dd, $J$=9.0, 5.8 Hz, 1H), 4.55 (dd, $J$=8.3, 6.2 Hz, 1H), 4.42 (t, $J$=7.8 Hz, 1H), 4.19 (m, 1H), 4.00 (m, 1H), 3.52-3.36 (m, 2H), 3.09 (m, 2H), 3.02-2.83 (m, 5H), 2.78 (m, 1H), 1.81 (m, 1H), 1.65 (m, 3H), 1.31-1.18 (m, 2H). $^{13}$C NMR (125 MHz, Methanol-$d_4$) δ 175.46, 172.51, 172.03, 170.89, 167.01, 152.28, 141.24, 136.22, 136.10, 135.59, 133.68, 132.98, 132.01, 131.79, 128.58, 128.03, 127.72, 127.31, 127.08, 126.86, 126.78, 126.71, 126.45, 126.00, 125.30, 124.90, 124.84, 122.85, 120.87, 120.63, 118.08, 117.45, 116.70, 113.38, 110.61, 108.92, 55.39, 54.84, 53.98, 52.57, 41.59, 36.41, 36.02, 29.94, 26.78, 26.50, 24.70, 22.23. HRMS (ESI): Calculated for $C_{51}H_{56}N_9O_5S[M+H]^+$: 906.4120, Found: 906.4116.

Example 58 Preparation of Compound **2-58**

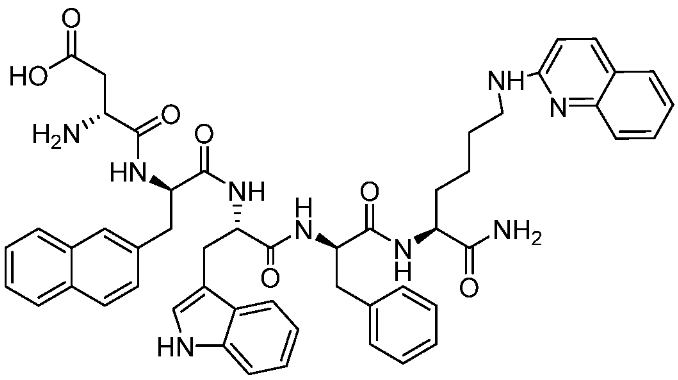

**2-58**

Compound 2-58 was obtained by the same preparation method in example 1 except that 2-(2,2-dimethoxyethylthio)quinoline or 2-(2,2-dimethoxyethylseleno)quinoline was used and the nucleophile was replaced with aspartic acid-pralmorelin, with a yield of 93%-97%. $^1$H NMR (500 MHz, Methanol-$d_4$) δ 7.76 (dd, $J$=9.2, 3.6 Hz, 2H), 7.71-7.60 (m, 3H), 7.57 (dd, $J$=7.9, 1.5 Hz, 1H), 7.52 (s, 1H), 7.48 (t, $J$=7.9 Hz, 2H), 7.40 (m, 2H), 7.35 (d, $J$=8.1 Hz, 1H), 7.33-7.13 (m, 6H), 7.13-7.09 (m, 2H), 7.04 (t, $J$=7.5 Hz, 1H), 6.99 (s, 1H), 6.73 (d, $J$=9.0 Hz, 1H), 4.56 (dd, $J$=8.1, 5.9 Hz, 1H), 4.52-4.41 (m, 2H), 4.18 (m, 1H), 3.57 (dd, $J$=7.6, 5.4 Hz, 1H), 3.37 (m, 2H), 3.19 (m, 1H), 3.11 (m, 1H), 3.08-2.92 (m, 3H), 2.90-2.82 (m, 1H), 2.56 (m, 1H), 2.35 (m, 1H), 1.80 (m, 1H), 1.58 (m, 3H), 1.26-1.14 (m, 2H). $^{13}$C NMR (125 MHz, Methanol-$d_4$) δ 177.48, 176.00, 175.80, 172.85, 172.32, 172.16, 157.59, 147.62, 136.78, 136.62, 134.30, 133.48, 132.45, 129.00, 128.15, 127.64, 127.52, 127.36, 127.31, 127.21, 127.11, 126.48, 125.58, 125.17, 124.44, 123.33, 123.15, 121.32, 121.07, 118.50, 117.91, 112.66, 111.04, 109.49, 55.53, 55.04, 54.78, 53.42, 52.56, 42.65, 40.65, 37.26, 36.66, 30.96, 28.48, 26.86, 23.05. HRMS (ESI): Calculated for $C_{52}H_{54}N_9O_7$ [M-H]-: 916.4152, Found: 916.4158.

Example 59 Preparation of Compound **2-59**

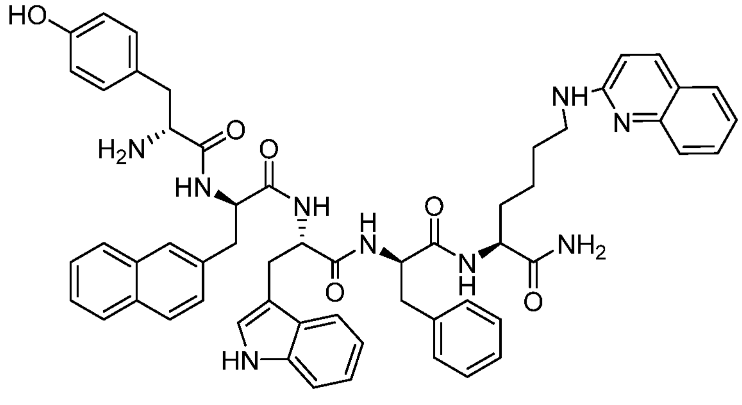

**2-59**

Compound 2-59 was obtained by the same preparation method in example 1 except that 2-(2,2-dimethoxyethylthio)quinoline or 2-(2,2-dimethoxyethylseleno)quinoline was used and the nucleophile was replaced with tyrosine-pralmorelin, with a yield of 69%-90%. $^1$H NMR (500 MHz, DMSO-$d_6$) δ 10.83 (d, *J*=2.4 Hz, 1H), 9.18 (s, 1H), 8.57 (d, *J*=8.0 Hz, 1H), 8.44 (d, *J*=8.1 Hz, 1H), 8.14 (d, *J*=8.4 Hz, 1H), 7.93 (d, *J*=8.2 Hz, 1H), 7.82-7.69 (m, 3H), 7.58 (dd, *J*=7.9, 1.4 Hz, 1H), 7.49 (d, *J*=8.2 Hz, 1H), 7.47-7.42 (m, 1H), 7.41-7.36 (m, 2H), 7.36-7.30 (m, 5H), 7.29-7.23 (m, 3H), 7.21 (d, *J*=2.4 Hz, 1H), 7.19-7.06 (m, 4H), 7.00 (s, 2H), 6.95-6.88 (m, 3H), 6.74 (d, *J*=8.9 Hz, 1H), 6.66 (m, 3H), 4.71 (m, 1H), 4.62 (m, 1H), 4.52 (m, 1H), 4.19 (m, 1H), 4.11 (m, 1H), 3.19 (d, *J*=3.8 Hz, 2H), 3.01 (m, 1H), 2.89-2.76 (m, 3H), 2.75-2.65 (m, 3H), 2.39 (m, 1H), 1.67 (m, 1H), 1.52 (m, 3H), 1.24 (m, 2H). $^{13}$C NMR (125 MHz, Methanol-$d_4$) δ 174.03, 173.61, 172.24, 171.30, 170.49, 157.45, 156.33, 148.43, 138.19, 136.66, 136.45, 135.01, 133.10, 132.09, 130.66, 129.88, 129.35, 128.55, 128.42, 128.01, 127.86, 127.70, 127.61, 127.31, 126.74, 126.01, 125.65, 124.71, 123.23, 121.37, 119.34, 118.73, 115.51, 113.57, 111.82, 110.54, 56.34, 54.73, 53.97, 53.05, 52.60, 40.76, 38.75, 38.43, 32.21, 28.95, 28.45, 23.34. HRMS (ESI): Calculated for $C_{57}H_{60}N_9O_6[M+H]^+$: 966.4661, Found: 966.4668.

Example 60 Preparation of Compound **2-60**

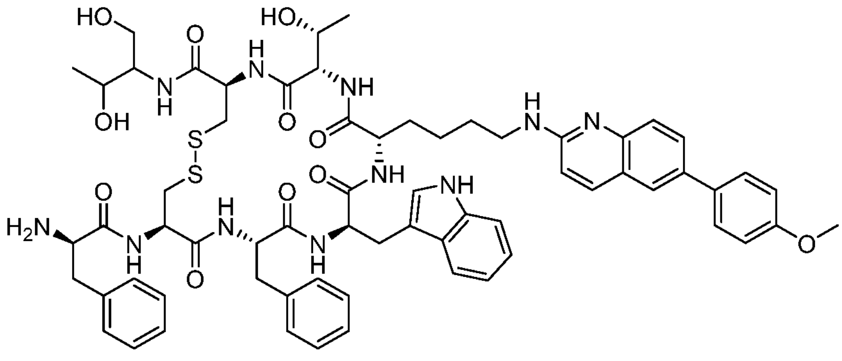

**2-60**

Compound 2-60 was obtained by the same preparation method in example 1 except that 6-p-methoxyphenyl-2-(2,2-dimethoxyethylthio)quinoline or 6-p-methoxyphenyl-2-(2,2-dimethoxyethylseleno)quinoline was used and the nucleophile was replaced with octreotide acetate hydrochloride, with a yield of 39%-46%. $^1$H NMR (600 MHz, Methanol-$d_4$) δ 7.87 (d, *J*=8.9 Hz, 1H), 7.77 (d, *J*=2.2 Hz, 1H), 7.76-7.72 (m, 1H), 7.70 (d, *J*=8.6 Hz, 1H), 7.63-7.58 (m, 2H), 7.43 (dd, *J*=7.7, 1.3 Hz, 1H), 7.34 (d, *J*=8.1 Hz, 1H), 7.32-7.28 (m, 4H), 7.26 (m, 3H), 7.24-7.21 (m, 3H), 7.04 (m, 2H), 7.01-6.99 (m, 2H), 6.93 (s, 1H), 6.74 (m, 1H), 5.19-5.12 (m, 1H), 5.11-5.05 (m, 1H), 4.64 (m, 1H), 4.43 (d, *J*=5.2 Hz, 1H), 4.39-4.35 (m, 1H), 4.14 (m, 1H), 4.05 (dd, *J*=6.5, 3.3 Hz, 1H), 3.99 (m, 1H), 3.85-3.81 (m, 4H), 3.74-3.62 (m, 3H), 3.12-2.75 (m, 10H), 1.76 (m, 1H), 1.48-1.32 (m, 3H), 1.26 (d, *J*=6.4 Hz, 3H), 1.18 (d, *J*=6.5 Hz, 3H), 0.79-0.62 (m, 2H).$^{13}$C NMR (150 MHz, Methanol-$d_4$) δ 174.82, 174.40, 174.16, 171.44, 171.37, 171.33, 169.01, 158.98, 157.40, 146.50, 137.27, 136.99, 136.55, 136.46, 134.12, 133.04, 129.02, 128.96, 128.13, 128.02, 127.95, 127.34, 126.93, 126.39, 126.35, 124.56, 124.25, 123.25, 123.02, 121.05, 118.41, 117.71, 113.79, 112.98,111.01, 108.47, 66.86, 65.62, 61.18, 59.69, 56.28, 56.16, 55.96, 54.85, 54.24, 54.18, 52.41, 52.33, 44.23, 41.78, 40.82, 40.58, 39.17, 30.27, 28.43, 25.72, 22.61, 18.90, 18.69. HRMS (ESI): Calculated for $C_{65}H_{78}N_{11}O_{11}S_2$ $[M+H]^+$: 1252.5318, Found: 1252.5313.

Example 61 Preparation of Compound **2-61**

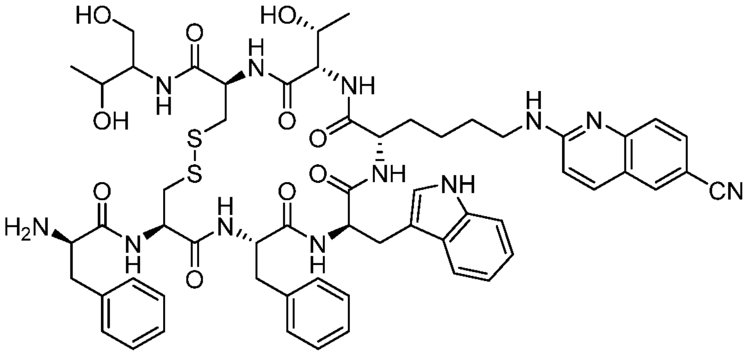

**2-61**

Compound 2-61 was obtained by the same preparation method in example 1 except that 6-cyano-2-(2,2-dimethoxyethylthio)quinoline or 6-cyano-2-(2,2-dimethoxyethylseleno)quinoline was used and the nucleophile was replaced with octreotide acetate hydrochloride, with a yield of 58%. $^1$H NMR (500 MHz, Methanol-$d_4$) δ 8.01 (d, *J*=1.8 Hz, 1H), 7.85 (d, *J*=9.3 Hz, 1H), 7.71-7.66 (m, 2H), 7.46-7.43 (m, 1H), 7.34-7.25 (m, 11H), 7.10-7.02 (m, 2H), 6.95 (s, 1H), 6.82 (d, *J*=9.1 Hz, 1H), 5.16 (m, 2H), 4.67 (m, 1H), 4.46 (d, *J*=5.3 Hz, 1H), 4.42-4.35 (m, 1H), 4.17 (m, 1H), 4.08 (dd, *J*=6.5, 3.2 Hz, 1H), 4.02 (m, 1H), 3.84 (dd, *J*=6.1, 3.3 Hz, 1H), 3.81 (m, 1H), 3.73 (m, 1H), 3.67 (m, 1H), 3.13 (m, 2H), 3.07-2.85 (m, 7H), 2.80 (m, 1H), 1.79 (m, 1H), 1.50-1.36 (m, 3H), 1.28 (d, *J*=6.3 Hz, 3H), 1.21 (d, *J*=6.5 Hz, 3H), 0.81-0.68 (m, 2H). $^{13}$C NMR (125 MHz, Methanol-$d_4$) δ 174.45, 174.15, 173.81, 171.49, 171.43, 171.38, 169.11, 158.99, 150.30, 140.19, 136.76, 136.65, 136.54, 136.37, 133.04, 130.53, 129.15, 129.04, 128.35, 128.05, 127.04, 126.68, 126.51, 125.77, 123.15, 122.75, 121.16, 119.09, 118.53, 117.83, 116.37, 111.09, 108.63, 103.50, 67.00, 65.79, 61.30, 59.75, 56.35, 56.25, 55.75, 54.96, 54.18, 52.67, 44.36, 42.08, 40.45, 40.32, 39.24, 30.31, 28.29, 25.85, 22.64, 19.00, 18.79. HRMS (ESI): Calculated for $C_{59}H_{71}N_{12}O_{10}S_2$ [M+H]$^+$: 1171.4852, Found: 1171.4837.

Example 62 Preparation of Compound **2-62**

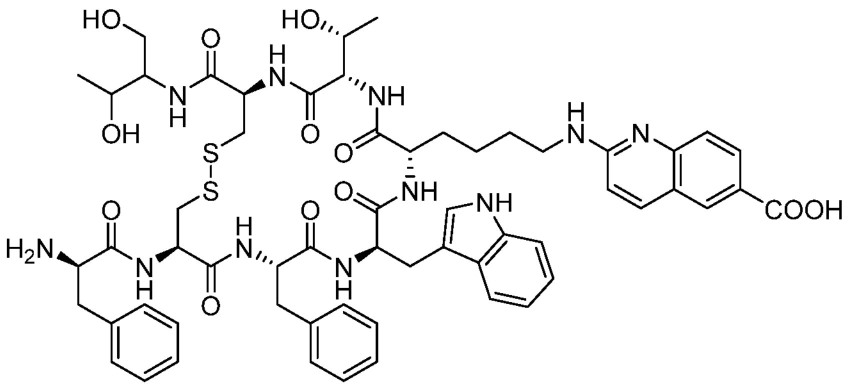

**2-62**

Compound 2-62 was obtained by the same preparation method in example 1 except that 6-carboxyl-2-(2,2-dimethoxyethylthio)quinoline was used and the nucleophile was replaced with octreotide acetate hydrochloride, with a yield of 60%. $^1$H NMR (500 MHz, Methanol-$d_4$) δ 8.22 (d, *J*=2.0 Hz, 1H), 8.10 (dd, *J*=8.6, 2.1 Hz, 1H), 7.87 (d, *J*=9.0 Hz, 1H), 7.61 (d, *J*=8.7 Hz, 1H), 7.43 (d, *J*=7.6 Hz, 1H), 7.28 (m, 11H), 7.07-7.00 (m, 2H), 6.94 (s, 1H), 6.73 (d, *J*=9.0 Hz, 1H), 5.12 (m, 1H), 5.05 (dd, *J*=7.4, 3.5 Hz, 1H), 4.65 (m, 1H), 4.43 (d, *J*=5.0 Hz, 1H), 4.42-4.36 (m, 1H), 4.14 (m, 1H), 4.05 (dd, *J*=6.5, 3.3 Hz, 1H), 3.96 (m, 1H), 3.83 (dd, *J*=6.2, 3.3 Hz, 1H), 3.72 (m, 1H), 3.69-3.61 (m, 2H), 3.21-2.72 (m, 10H), 1.74 (m, 1H), 1.38 (m, 3H), 1.26 (d, *J*=6.3 Hz, 3H), 1.19 (d, *J*=6.5 Hz, 3H), 0.78-0.59 (m, 2H).$^{13}$C NMR (125 MHz, Methanol-$d_4$) δ 175.10, 174.54, 174.33, 173.93, 171.50, 171.43, 169.02, 158.13, 149.03, 137.65, 137.46, 136.60, 136.52, 130.94, 130.09, 129.07, 129.03, 128.96, 128.17, 128.03, 126.99, 126.47, 126.36, 123.40, 123.08, 122.12, 121.11, 118.46, 117.75, 112.78, 111.09, 108.51, 66.88, 65.70, 61.25, 59.81, 56.37, 56.27, 56.10, 54.94, 54.36, 52.40, 52.38, 44.17, 41.59, 41.00, 40.62, 39.28, 30.32, 28.43, 25.81, 22.69, 18.97, 18.76. HRMS (ESI): Calculated for $C_{59}H_{72}N_{11}O_{12}S_2$ [M+H]$^+$: 1190.4798, Found: 1190.4801.

Example 63 Preparation of Compound **2-63**

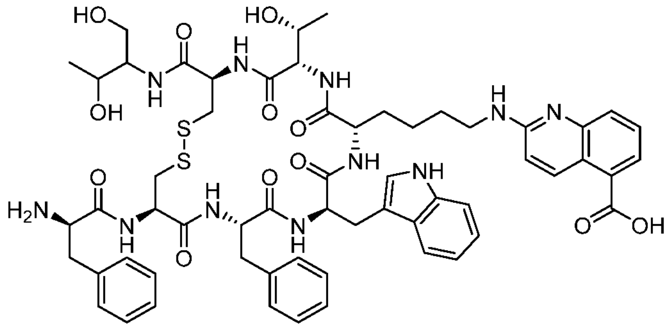

**2-63**

Compound 2-63 was obtained by the same preparation method in example 1 except that 5-carboxyl-2-(2,2-dimethoxyethylthio)quinoline was used and the nucleophile was replaced with octreotide acetate hydrochloride, with a yield of 12%. $^{1}$H NMR (600 MHz, Methanol-$d_4$) δ 8.55 (m, 1H), 7.66-7.63 (m, 1H), 7.44 (d, *J*=4.8 Hz, 2H), 7.42 (d, *J*=7.7 Hz, 1H), 7.30 (m, 7H), 7.26-7.23 (m, 4H), 7.07-7.00 (m, 2H), 6.93 (s, 1H), 6.72 (d, *J*=9.3 Hz, 1H), 5.11 (m, 1H), 5.04 (m, 1H), 4.65 (m, 1H), 4.42 (d, *J*=5.1 Hz, 1H), 4.40-4.35 (m, 1H), 4.12 (m, 1H), 4.04 (m, 1H), 3.94 (m, 1H), 3.83 (m, 1H), 3.71 (m, 1H), 3.65 (m, 2H), 3.26 (m, 2H), 3.11 (m, 1H), 3.05-2.89 (m, 7H), 2.83-2.75 (m, 2H), 1.78-1.66 (m, 1H), 1.43-1.31 (m, 3H), 1.26 (d, *J*=6.4 Hz, 3H), 1.18 (d, *J*=6.4 Hz, 3H), 0.73-0.57 (m, 2H). $^{13}$C NMR (150 MHz, Methanol-$d_4$) δ 175.38, 174.65, 174.11, 173.93, 171.09, 171.02, 170.99, 168.58, 156.77, 151.77, 147.20, 138.14, 137.03, 136.18, 136.12, 135.48, 128.65, 128.63, 127.75, 127.61, 126.57, 126.05, 125.95, 124.64, 122.67, 120.69, 120.60, 119.83, 118.05, 117.32, 111.98, 110.68, 108.08, 66.45, 65.27, 60.82, 59.39, 55.95, 55.88, 55.67, 54.52, 53.99, 51.99, 51.95 , 43.77, 41.15, 40.57, 40.19, 38.88, 29.91, 28.09, 25.40, 22.28, 18.55, 18.34. HRMS (ESI): Calculated for $C_{59}H_{72}N_{11}O_{12}S_2$[M+H]$^+$: 1190.4798, Found: 1190.4831.

Example 64 Preparation of Compound 2-64

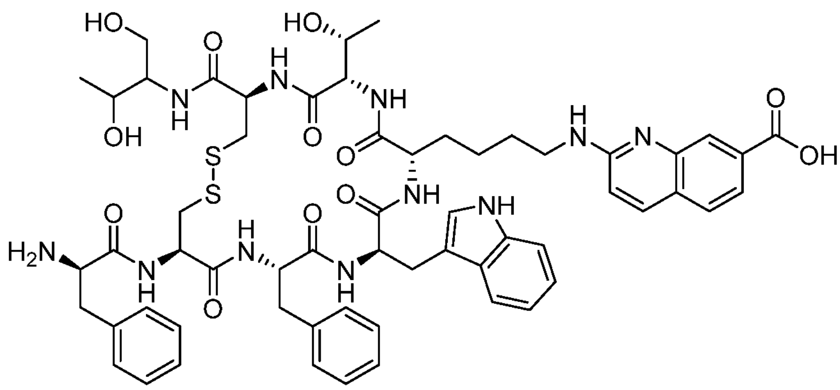

**2-64**

Compound 2-64 was obtained by the same preparation method in example 1 except that 8-carboxyl-2-(2,2-dimethoxyethylthio)quinoline was used and the nucleophile was replaced with octreotide acetate hydrochloride, with a yield of 59%. $^{1}$H NMR (500 MHz, Methanol-$d_4$) δ 8.32 (d, *J*=1.5 Hz, 1H), 7.84 (d, *J*=9.0 Hz, 1H), 7.77 (dd, *J*=8.2, 1.7 Hz, 1H), 7.58 (d, *J*=8.2 Hz, 1H), 7.43 (d, *J*=7.9 Hz, 1H), 7.35-7.24 (m, 11H), 7.08-7.00 (m, 2H), 6.98 (s, 1H), 6.75 (d, *J*=9.0 Hz, 1H), 5.12 (m, 1H), 5.05 (m, 1H), 4.70-4.67 (m, 1H), 4.46-4.39 (m, 2H), 4.15 (m, 1H), 4.07 (m, 1H), 3.95 (m, 1H), 3.86 (m, 1H), 3.74 (m, 1H), 3.71-3.64 (m, 2H), 3.31 (d, *J*=7.2 Hz, 2H), 3.15 (m, 1H), 3.07-2.81 (m, 9H), 1.73 (m, 1H), 1.45-1.36 (m, 3H), 1.29 (d, *J*=6.2 Hz, 3H), 1.21 (d, *J*=6.4 Hz, 3H), 0.77-0.60 (m, 2H). $^{13}$C NMR (125 MHz, Methanol-$d_4$) δ 175.04, 174.59, 174.45, 174.23, 171.59, 171.48, 171.45, 169.00, 157.69, 147.25, 139.06, 137.44, 136.63, 136.53, 136.45, 129.08, 128.20, 128.06, 127.04, 126.50, 126.45, 126.41, 125.68, 124.30, 123.17, 122.14, 121.12, 118.47, 117.75, 113.28, 111.20, 108.51, 66.87, 65.76, 61.28, 59.89, 56.42, 56.37, 56.11, 54.97, 54.53, 52.41, 44.13, 41.44, 40.98, 40.67, 39.32, 30.37, 28.49, 25.88, 22.73, 19.01, 18.81.HRMS (ESI): Calculated for $C_{59}H_{72}N_{11}O_{12}S_2$[M+H]$^+$: 1190.4798, Found: 1190.4814.

Example 65 Preparation of Compound **2-65**

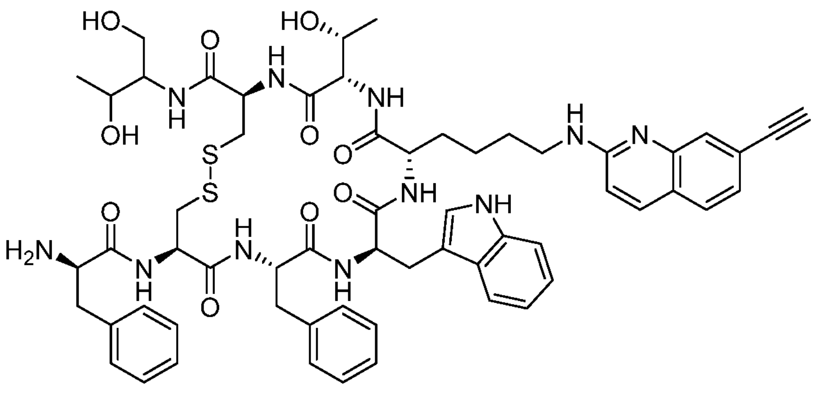

**2-65**

Compound 2-65 was obtained by the same preparation method in example 1 except that 7-alkynyl-2-(2,2-dimethoxyethylthio)quinoline was used and the nucleophile was replaced with octreotide acetate hydrochloride, with a yield of 32%. $^1$H NMR (500 MHz, Methanol-$d_4$) δ 7.81 (d, *J*=9.0 Hz, 1H), 7.79 (d, *J*=1.4 Hz, 1H), 7.57 (d, *J*=8.1 Hz, 1H), 7.47-7.43 (m, 1H), 7.34-7.25 (m, 12H), 7.06 (m, 2H), 6.96 (s, 1H), 6.75 (d, *J*=9.0 Hz, 1H), 5.18 (m, 1H), 5.11 (m, 1H), 4.70-4.67 (m, 1H), 4.46 (d, *J*=5.2 Hz, 1H), 4.40 (d, *J*=6.0 Hz, 1H), 4.20-4.16 (m, 1H), 4.07 (dd, *J*=6.5, 3.3 Hz, 1H), 4.00 (m, 1H), 3.86 (m, 1H), 3.76-3.70 (m, 2H), 3.67 (d, *J*=5.0 Hz, 1H), 3.57 (s, 1H), 3.29 (m, 2H), 3.12-2.85 (m, 10H), 1.77 (m, 1H), 1.49-1.35 (m, 3H), 1.28 (d, *J*=6.3 Hz, 3H), 1.21 (d, *J*=6.5 Hz, 3H), 0.81-0.64 (m, 2H). $^{13}$C NMR (125 MHz, Methanol-$d_4$) δ 174.72, 174.47, 174.23, 171.46, 171.42, 169.09, 157.96, 147.25, 137.26, 136.64, 136.55, 136.37, 129.11, 129.06, 128.23, 128.09, 128.05, 127.42, 127.02, 126.48, 124.27, 123.25, 123.13, 121.15, 118.51, 117.80, 113.68, 111.09, 108.58, 83.34, 77.89, 66.95, 65.75, 61.29, 59.77, 56.38, 56.27, 56.01, 54.95, 54.28, 52.53, 52.43, 44.33, 41.88, 40.82, 40.58, 39.27, 30.36, 28.37, 25.84, 22.69, 18.99, 18.79. HRMS (ESI): Calculated for $C_{60}H_{72}N_{11}O_{10}S_2$ [M+H]$^+$: 1170.49, Found: 1170.4897.

Example 66 Preparation of Compound **2-66**

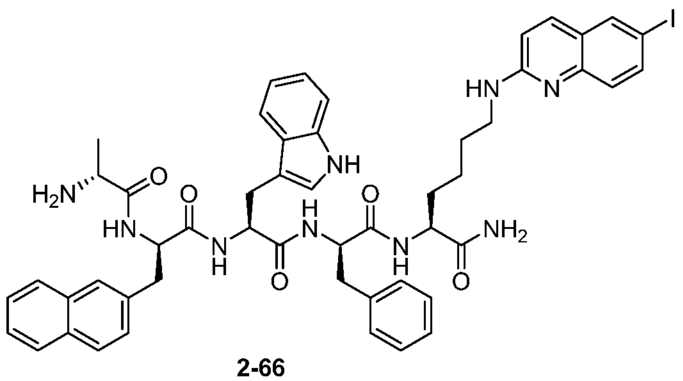

**2-66**

Compound 2-66 was obtained by the same preparation method in example 1 except that 7-iodo-2-(2,2-dimethoxyethylthio)quinoline or 7-iodo-2-(2,2-dimethoxyethylseleno)quinoline was used and the nucleophile was replaced with pralmorelin, with a yield of 27%-36%. $^1$H NMR (500 MHz, Methanol-$d_4$) δ 7.94 (d, *J*=2.1 Hz, 1H), 7.83-7.75 (m, 1H), 7.73-7.65 (m, 4H), 7.54 (d, *J*=1.6 Hz, 1H), 7.48-7.45 (m, 1H), 7.45-7.42 (m, 2H), 7.39 (d, *J*=8.8 Hz, 1H), 7.35 (d, *J*=8.1 Hz, 1H), 7.28-7.21 (m, 3H), 7.20-7.16 (m, 1H), 7.13 (m, 3H), 7.05 (m, 1H), 6.93 (s, 1H), 6.74 (d, *J*=9.0 Hz, 1H), 4.63 (dd, *J*=8.6, 6.0 Hz, 1H), 4.58 (dd, *J*=7.8, 6.3 Hz, 1H), 4.41 (dd, *J*=8.5, 7.0 Hz, 1H), 4.19-4.10 (m, 1H), 3.61 (q, *J*=7.0 Hz, 1H), 3.39 (dd, *J*=14.5, 7.2 Hz, 2H), 3.10 (,m 2H), 2.99 (dd, *J*=13.8, 8.5 Hz, 1H), 2.93 (dd, *J*=14.1, 8.1 Hz, 2H), 2.76 (dd, *J*=13.4, 7.1 Hz, 1H), 1.87-1.75 (m, 1H), 1.63-1.48 (m, 3H), 1.28 (d, *J*=7.0 Hz, 3H), 1.21-1.10 (m, 2H). $^{13}$C NMR (125 MHz, Methanol-$d_4$) δ 175.97 , 172.77 , 172.56, 172.40, 171.42 , 157.74 , 146.87, 137.52 , 136.59 , 136.49, 135.82 , 135.56 , 134.16 , 133.45 , 132.49 , 128.94 , 128.18 , 127.68 , 127.53 , 127.38 , 127.19, 127.17 , 126.97 , 126.56 , 125.71 , 125.30 , 125.19, 123.24 , 121.09 , 118.54 , 117.85 , 113.53, 111.03 , 109.26 , 55.73 , 54.88 , 54.23 , 53.30 , 49.14 , 40.52 , 37.11 , 36.53 , 30.77 , 28.35 , 27.25 , 22.99 , 17.50 . HRMS (ESI): Calculated for $C_{51}H_{55}IN_9O_5$ [M+H]$^+$: 1000.3365, Found: 1000.3354.

Example 67 Preparation of Compound **2-67**

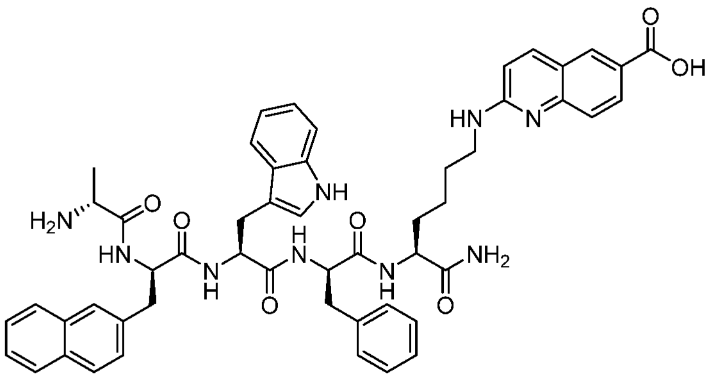

**2-67**

Compound 2-67 was obtained by the same preparation method in example 1 except that 7-carboxyl-2-(2,2-dimethoxyethylthio)quinoline was used and the nucleophile was replaced with pralmorelin, with a yield of 77%. $^1H$ NMR (500 MHz, Methanol-$d_4$) δ 8.32 (s, 1H), 8.13 (d, *J*=8.8 Hz, 1H), 7.94 (d, *J*=9.1 Hz, 1H), 7.83-7.76 (m, 1H), 7.74-7.65 (m, 3H), 7.57 (s, 1H), 7.48 (d, *J*=7.9 Hz, 1H), 7.44 (p, *J*=5.4 Hz, 2H), 7.34 (d, *J*=8.1 Hz, 1H), 7.26 (m, 3H), 7.19 (d, *J*=7.3 Hz, 1H), 7.14 (d, *J*=7.5 Hz, 3H), 7.04 (d, *J*=7.4 Hz, 1H), 6.94 (s, 1H), 6.85 (d, *J*=9.1 Hz, 1H), 4.68-4.64 (m, 1H), 4.60 (dd, *J*=7.9, 6.5 Hz, 1H), 4.41 (dd, *J*=8.6, 7.0 Hz, 1H), 4.15 (dd, *J*=10.2, 4.2 Hz, 1H), 3.81 (q, *J*=7.1 Hz, 1H), 3.43 (m2H), 3.10 (m, 2H), 3.03-2.98 (m, 1H), 2.97-2.89 (m, 2H), 2.78-2.71 (m, 1H), 1.89-1.75 (m, 1H), 1.59 (m, 3H), 1.38 (d, *J*=7.1 Hz, 3H), 1.17 (m, 2H). $^{13}C$ NMR (125 MHz, Methanol-$d_4$) δ 176.01 , 172.81, 172.46, 171.14, 170.02, 136.57, 136.46, 134.15, 133.45 , 132.49 , 130.21 , 128.93 , 128.17 , 127.72 , 127.51 , 127.36 , 127.16 , 126.91 , 126.57 , 125.74 , 125.33 , 123.21 , 121.86, 121.07 , 118.51 , 117.86 , 111.02 , 109.31 , 55.78 , 55.05 , 54.15 , 53.22 , 48.68 , 40.80 , 36.96 , 36.45 , 30.61 , 28.01 , 27.34 , 22.90 , 16.15 . HRMS (ESI): Calculated for $C_{52}H_{56}N_9O_7$ $[M+H]^+$: 918.4297, Found: 918.4318.

Example 68 Preparation of Compound **2-68** (H-His7-Aib8-Glu9-Gly10-Thr11-Phe12-Thr13-Ser14-Asp15-Val16-Ser17-Ser18-Tyr19-Leu20-Glu21-Gly22-Gln23-Ala24-Ala25-Lys26(quinolinyl)-Glu27-Phe28-Ile29-Ala30-Trp31-Leu32-Val33-Arg34-Gly35-Arg36-Gly37-OH)

Compound 2-68 was obtained by the same preparation method in example 1 except that 2-(2,2-dimethoxyethylthio)quinoline or 2-(2,2-dimethoxyethylseleno)quinoline was used and the nucleophile was replaced with semaglutide backbone (H-His7-Aib8-Glu9-Gly10-Thr11-Phe12-Thr13-Ser14-Asp15-Val16-Ser17-Ser18-Tyr19-Leu20-Glu21-Gly22-Gln23-Ala24-Ala25-Lys26-Glu27-Phe28-Ile29-Ala30-Trp31-Leu32-Val3 3-Arg34-Gly35-Arg36-Gly37-OH). The structure of the product was identified by biological mass spectrum (Fig. 1), and the molecular weight 3522.7392 is the molecular weight of the quinolinated semaglutide backbone product.

Example 69 Preparation of Compound **2-69** (quinolinated insulin)

Compound 2-69 was obtained by the same preparation method in example 1 except that 2-(2,2-dimethoxyethylthio)quinoline or 2-(2,2-dimethoxyethylseleno)quinoline was used and the nucleophile was replaced with insulin. The structure of the product was identified by the biological mass spectrum (Fig. 2), and the molecular weight 5930.6892 is the molecular weight of the quinolinated insulin product.

**Biological test example 1** (cell proliferation inhibition assay):

Hepatocarcinoma BEL-7402 and SMMC-7721 cells were purchased from American Standard Bacterial Bank. All cell lines were stored in a cell culture medium containing 10% fetal bovine serum in a humid environment at 37 °C and 5% $CO_2$. The two kinds of hepatocarcinoma cells were seeded in 96-well plates overnight. The assay were performed using a final highest concentration of 100 μM, 10-fold dilution, 6 gradients and 3 duplicates, and the cells were treated for 72 hours and three independent repeated experiments were carried out. The antiproliferative activity of the compound was determined by Sulforhodamine B (SRB) method. Then the absorbance OD value at a wavelength of 510 nm was measured on a Synergy H4 Hybrid reader (BioTek, Winooski, VT, USA) by using Gen5.0 software (BioTek). The $IC_{50}$ value was calculated using the software Prism 5 (GraphPad Software, Inc). The test results are shown in Table 1.

Table 1 Hepatocarcinoma cell proliferation inhibition results of selected compounds

| Compound name/No. | Proliferation inhibition $IC_{50}$ (μM) | |
|---|---|---|
| | BEL-7402 | SMMC-7721 |
| YSL | >100 | >100 |
| YSL-M | >100 | >100 |

(continued)

| Compound name/No. | Proliferation inhibition $IC_{50}$ (μM) | |
|---|---|---|
| | BEL-7402 | SMMC-7721 |
| 2-39 | 52.57±2.47 | 63.30±6.35 |

Tyroserleutide (YSL) has already been in a phase III clinical trial for treatment of liver cancer. YSL, YSL-M (tyroserleucide methyl ester) and the Compound 2-39 which is modified with quinolinyl group were tested for their anti-cancer activities on hepatocarcinoma BEL-7402 and SMMC-7721 cells. The results shows that the Compound 2-39 has better cytotoxicities than the unmodified drugs on both BEL-7402 and SMMC-7721 cells, which indicates that the introduction of quinolinyl in a peptide can enhance its activity.

**Biological test example 2** ([$^{35}$S]GTPγS binding assay):

Protein concentration was detected using BCA protein concentration assay kit: 10 μl of protein standard was prepared into a final concentration of 0.5 mg/ml. 0, 1, 2, 4, 8, 12, 16, or 20 μl of BSA standard protein was taken and added together with the sample to be tested to a 96-well plate, and supplemented to 20 μl with the solution for diluting the standard. 200 μl of BCA working solution was added to each well and placed at 37 °C for 30 min. The absorbance at A562 wavelength was measured with a microplate reader. The protein concentration was calculated based on the standard curve.

The prepared membrane receptor was diluted to the required concentration with the reaction buffer (R.B), and the samples were added as shown in Table 2 (unit: μl).

Table 2

| | R.B | [$^{35}$S]GTPγS 0.1-0.2 nM | GTPγS 20 μM | GDP 40 μM | agonist | protein 20-30 μg |
|---|---|---|---|---|---|---|
| NS binding | 30 | 10 | 10 | 0 | 0 | 50 |
| basal | 30 | 10 | 0 | 10 | 0 | 50 |
| agonist | 20 | 10 | 0 | 10 | 10 | 50 |

The reaction tube was incubated in a 27 °C water bath for 1 hour, and the mixture was filtered through a glass fiber membrane under reduced pressure and liquid scintillation counting was performed. The following equation was used to perform calculation:

$$[^{35}S]\ GTP_{\gamma}S\ \text{binding rate} = 100\times(cpm_{sample} - cpm_{non\text{-}specific})/(cpm_{basal} - cpm_{non\text{-}specific})$$

The μ-opioid receptor (MOR) agonist activities of derorphin and its quinolinylated compound 2-38 are shown in Table 3 below.

Table 3 μ-Opioid Receptor (MOR) Agonist Activity Assay

| Compound name/No | (MOR) (mean±sem) | |
|---|---|---|
| | $EC_{50}$(nM) | Emax(%) |
| derorphin | 61.3±10.6 | 218.8±7.1 |
| **2-38** | 223.9±25.1 | 202.7±5.1 |

Derorphin is a clinically used μ-opioid receptor (MOR) agonist. From the above results in Table 3, it can be seen that the quinolinylated compound 2-38 has an activity comparable to that of derorphin.

**Biological test example 3** (liver tissue metabolism assay):

5 g of a fresh pig liver from which adhering fat and connective tissue were removed, was washed with a cold saline, absorbed water on its surface with a filter paper and weighed. The liver was cut into pieces with a scissors, added with an appropriate amount of a PBS buffer, and homogenized with a homogenizer at a frequency of 30/s for 4 minutes to prepare 20 ml of a slurry. The compound 2-40 to be tested (at a final concentration of 1 mg/ml with a co-solvent (1% DMSO)) was added into the 20 ml pig liver slurry, stirred thoroughly and left stand. 1ml of samples were withdrawn twice at six time points: 0.25 hour, 0.5 hour, 1 hour, 1.5 hour, 2 hour and 3 hour, respectively, and added with methanol (500

μl) to stop the reaction. After centrifugation (1000 rpm, 6 minutes), the supernatant was separated and extracted twice with 750 μl of water-saturated n-butanol. The extracted samples were then analyzed by HPLC. The HPLC analysis was performed on an Agilent Zorbax SB-C18 column (5 μm, 4.6x250 mm) with a flow rate of 1 mL/min at 25 °C. The gradient condition used was from 50% A (MeOH) and 50% B ($H_2O$ + 0.2% $CH_3COOH$) to 95% A and 5% B in 16 minutes. The absorbance values at 210, 254 and 280 nm were collected using a UV-DAD detector. The concentration of the compound 2-40 in liver tissue over time is shown in Fig. 3.

7-(2-fluorophenyl)-4-methylquinoline-2(1H)-one is a tankyrase (TNKS) inhibitor with an $IC_{50}$ value of 0.052 μM. It can be easily coupled to (S)-2-((S)-2-amino-3-methylbutamido)-N-(4-(hydroxymethyl)phenyl)-5-ureidopentanamide (Val-Cit-PAB-OH) by using the method developed in the present application to obtain the compound 2-40. As shown in Fig. 3, the compound 2-40 shows good metabolic stability in the in vitro liver tissue metabolic stability assay.

In conclusion, the present application provides a new bioorthogonal chemistry method for the preparation of small molecule-peptide coupled drugs. This method may be used in various applications and has great application potential in the modification of drugs, especially peptide drugs.

## Claims

**1.** A method for modifying or labeling the amino group(s) in the molecule(s) wherein the zolinium(s) represented by the following formula 1 is used to modify or label the amino group(s) in the molecule(s),

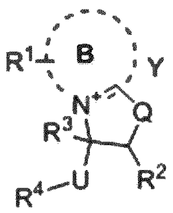

**1**

In the above formula 1,
Ring B represents a substituted or unsubstituted 5-, 6- or 7-membered nitrogen-containing heterocyclic ring;
Q and U each independently represent oxygen (O), sulfur (S), selenium (Se), tellurium (Te), polonium (Po), nitrogen (N), phosphorus (P), boron (B) or silicon (Si);
$R^1$ represents 1-5 substituents on the ring B, which are each independently selected from hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, and halogen; or, when $R^1$ represents two or more substituents on the ring B, two adjacent substituents of which may connect to each other together with the atoms on the ring B to form a substituted or unsubstituted aryl, a substituted or unsubstituted 3-7 membered cycloalkyl, a substituted or unsubstituted 5-7 membered heterocycloalkyl containing 1-5 atoms independently selected from oxygen (O), sulfur (S), selenium (Se), tellurium (Te), polonium (Po), nitrogen (N), phosphorus (P), boron (B) and silicon (Si), or a substituted or unsubstituted 5-7 membered heteroaryl containing 1-5 atoms independently selected from oxygen (O), sulfur (S), selenium (Se), tellurium (Te), polonium (Po), nitrogen (N), phosphorus (P), boron (B) and silicon (Si);
$R^2$ and $R^3$ each independently represent hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, or halogen;
$R^4$ represents hydrogen, a substituted or unsubstituted alkyl, or a substituted or unsubstituted aryl; and
Y is an acid anion selected from inorganic acid ions, organic acid ions and halide ions.

**2.** The method of claim 1, wherein,

in formula 1,
Ring B is a 5- or 6-membered ring;
Q and U are each independently selected from oxygen, sulfur and selenium;

$R^1$ represents two or more substituents on ring B, two adjacent substituents of which may connect to each other together with the atoms on the ring B to form a substituted or unsubstituted aryl ring;
$R^2$ and $R^3$ are each independently selected from hydrogen, substituted or unsubstituted alkyl, and substituted or unsubstituted aryl; and
$R^4$ is a substituted or unsubstituted alkyl.

3. The method of claim 1, wherein, the molecule includes amino acid esters, amino amides, peptides or proteins.

4. The method of claim 1, wherein, in the presence or absence of an additive, the zolinium(s) represented by Formula 1 reacts with the molecule(s) containing the amino group(s) in a solvent to prepare a product of which the amino group(s) is(are) labeled or modified.

5. The method of claim 4, wherein, the method is carried out by a reaction represented by the following reaction scheme I:

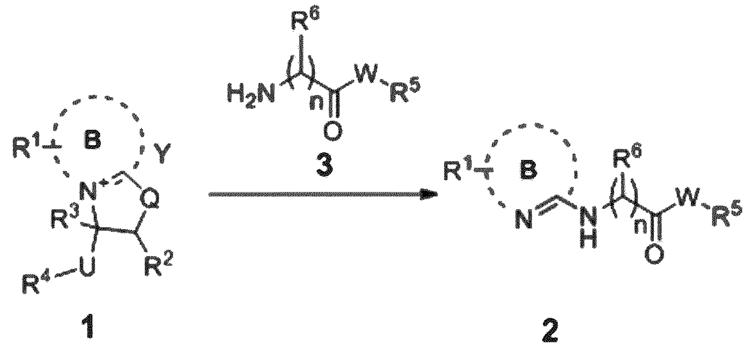

Reaction scheme I

In Reaction scheme I, in the presence or absence of an additive, the zolinium(s) represented by formula 1 reacts with the compound represented by formula 3 in a solvent to prepare a product represented by Formula 2 of which the amino group is modified,
wherein, in formula 3,
n represents an integer of 1-6;
$R^5$ represents hydrogen, a substituted or unsubstituted alkyl, or a substituted or unsubstituted aryl;
Each of $R^6$ independently represents hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, or halogen; or
$R^5$ and $R^6$ connect to each other to form a 5-7 membered heterocycle containing 1-3 heteroatoms selected from oxygen (O), sulfur (S), selenium (Se), tellurium (Te), polonium (Po), nitrogen (N), phosphorus (P), boron (B) and silicon (Si);
W represents oxygen (O), sulfur (S), selenium (Se), tellurium (Te), polonium (Po), nitrogen (N), phosphorus (P), boron (B), or silicon (Si).
wherein, in formula 2, ring B, $R^1$, $R^5$, $R^6$, W, and n are defined as those in formula 1 and formula 3.

6. The method of claim 5, wherein,

n is 1 or 5;
W is oxygen or nitrogen;
$R^5$ is hydrogen, or a substituted or unsubstituted alkyl; and
$R^6$ is amino, a substituted or unsubstituted amido, a substituted or unsubstituted alkyl, or a substituted or unsubstituted phenyl.

7. The method of claim 5, wherein, the solvent includes any one or a combination of two or more solvents of aprotic solvents and protic solvents, preferably, the solvent is n-butanol or water, and/or
the additive includes organic bases or inorganic bases.

**8.** Use of the zolinium(s) represented by the following formula 1 for modifying or labeling the amino group(s) in the molecule(s),

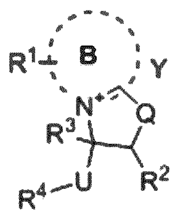

**1**

In the above formula 1,
Ring B represents a substituted or unsubstituted 5-, 6- or 7-membered nitrogen-containing heterocyclic ring;
Q and U each independently represent oxygen (O), sulfur (S), selenium (Se), tellurium (Te), polonium (Po), nitrogen (N), phosphorus (P), boron (B) or silicon (Si);
$R^1$ represents 1-5 substituents on the ring B, which are each independently selected from hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, and halogen; alternatively, when $R^1$ represents two or more substituents on the ring B, two adjacent substituents of which may connect to each other together with the atoms on the ring B to form a substituted or unsubstituted aryl, a substituted or unsubstituted 3-7 membered cycloalkyl, a substituted or unsubstituted 5-7 membered heterocycloalkyl containing 1-5 atoms independently selected from oxygen (O), sulfur (S), selenium (Se), tellurium (Te), polonium (Po), nitrogen (N), phosphorus (P), boron (B) and silicon (Si), or a substituted or unsubstituted 5-7 membered heteroaryl containing 1-5 atoms independently selected from oxygen (O), sulfur (S), selenium (Se), tellurium (Te), polonium (Po), nitrogen (N), phosphorus (P), boron (B) and silicon (Si);
$R^2$ and $R^3$ each independently represent hydrogen, hydroxyl, amino, mercapto, nitro, cyano, substituted or unsubstituted amide, substituted or unsubstituted alkyl, substituted or unsubstituted alkoxy, substituted or unsubstituted alkylthio, substituted or unsubstituted alkylamino, substituted or unsubstituted aryl, substituted or unsubstituted aryloxy, substituted or unsubstituted arylthio, substituted or unsubstituted arylamino, or halogen;
$R^4$ represents hydrogen, a substituted or unsubstituted alkyl, or a substituted or unsubstituted aryl;
Y is an acid anion selected from inorganic acid ions, organic acid ions and halide ions.

**9.** Use of claim 8, wherein, the molecule includes amino acid esters, amino amides, peptides or proteins.

**10.** Use of claim 8, wherein, the modifying or labeling is applied in fields of synthesizing pharmaceutical molecules, developing probe molecules and diagnostic labeling reagents.

Fig. 1

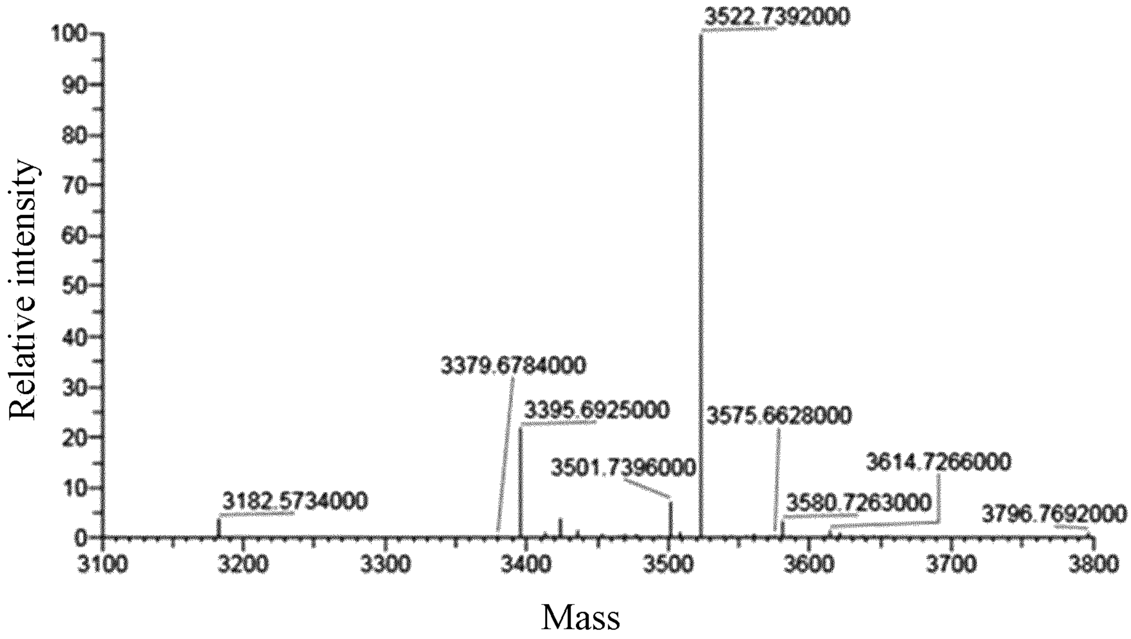
3522.7392000
3379.6784000
3395.6925000
3575.6628000
3501.7396000
3614.7266000
3182.5734000
3580.7263000
3796.7692000
100
90
80
70
60
50
40
30
20
10
0
Relative intensity
3100
3200
3300
3400
3500
3600
3700
3800
Mass

Fig. 2

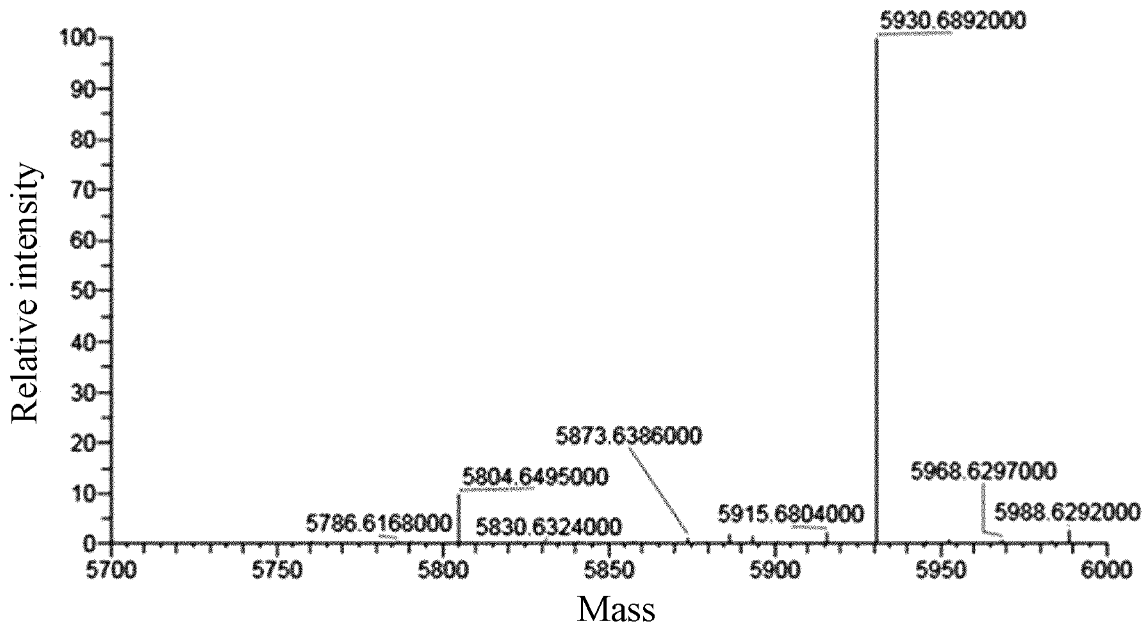
5930.6892000
5873.6386000
5968.6297000
5804.6495000
5915.6804000
5988.6292000
5786.6168000
5830.6324000
100
90
80
70
60
50
40
30
20
10
0
Relative intensity
5700
5750
5800
5850
5900
5950
6000
Mass

Fig. 3

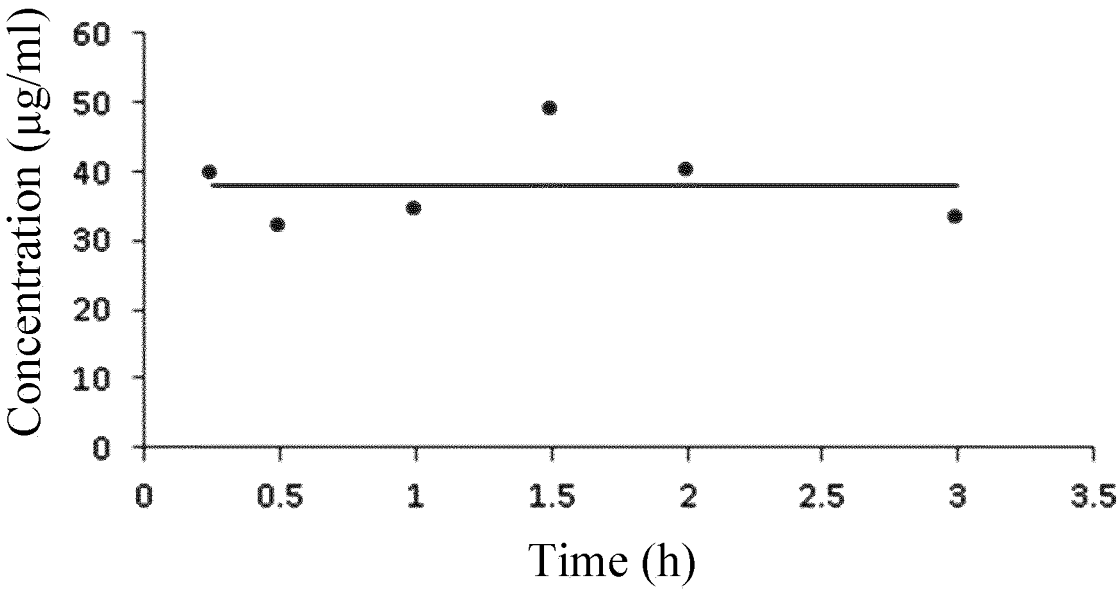

## INTERNATIONAL SEARCH REPORT

International application No.
**PCT/CN2020/081983**

**A. CLASSIFICATION OF SUBJECT MATTER**

C07D 498/04(2006.01)i; C07D 213/64(2006.01)i

According to International Patent Classification (IPC) or to both national classification and IPC

**B. FIELDS SEARCHED**

Minimum documentation searched (classification system followed by classification symbols)

C07D

Documentation searched other than minimum documentation to the extent that such documents are included in the fields searched

Electronic data base consulted during the international search (name of data base and, where practicable, search terms used)

CNABS, CNKI, VEN, CA, CASREACT: 中国科学院上海药物研究所, 喹啉, 唑啉, 氨基酸, 标记, quinoline, oxazole, amino acid

**C. DOCUMENTS CONSIDERED TO BE RELEVANT**

| Category* | Citation of document, with indication, where appropriate, of the relevant passages | Relevant to claim No. |
|---|---|---|
| PX | Peng Liu et al. "Metal-free quinolylation of the primary amino groups of amino acid derivatives and peptides with dihydrooxazolo[3, 2-a]quinoliniums" *Green Chemistry,* Vol. 21, No. 15, 12 July 2019 (2019-07-12), pp. 4231-4237 | 1-10 |
| X | Li, Bo et al. "Regioselectivity and Mechanism of Synthesizing N-Substituted 2-Pyridones and 2-Substituted Pyridines via Metal-Free C-O and C-N Bond-Cleaving of Oxazoline[3, 2-a]pyridiniums" *Scientific Reports,* No. 7, 25 January 2017 (2017-01-25), pp. 1-11 | 1-10 |
| A | CN 107759614 A (SHANGHAI INSTITUTE OF MATERIA MEDICA, CHINESE ACADEMY OF SCIENCES) 06 March 2018 (2018-03-06) entire document | 1-10 |

☐ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 June 2020** | **01 July 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 107759614 A


### Non-patent literature cited in the description


- **LI, BO et al.** initially discovered a new acetal rearrangement reaction and a novel class of N-substituted aryl isoquinolinone compounds with good anti-tumor activity prepared thereby. *Eur. J. Med. Chem.,* 2014, vol. 77, 204-210
- *Eur. J. Med. Chem.,* 2013, vol. 70, 677
- *Sci Rep,* 2017, vol. 7, 41287
- *Cancer Lett.,* 2018, vol. 421, 135-144
- **LI BO et al.** Discovery of N-substituted 3-arylisoquinolone derivatives as antitumor agents originating from O-substituted 3-arylisoquinolines via [2,3] or [3,3] rearrangement. *Eur. J. Med. Chem.,* 2014, vol. 77, 204-210
- **LI, BO et al.** Discovery of N-substituted 3-arylisoquinolone derivatives as antitumor agents originating from O-substituted 3-arylisoquinolines via [2,3] or [3,3] rearrangement. *Eur. J. Med. Chem.,* 2014, vol. 77, 204-210